# EUROPEAN PATENT APPLICATION

(11) **EP 2 354 794 A1**
(43) Date of publication of application: **10.08.2011**
(21) Application number: 10152103.7
(22) Date of filing: 29.01.2010
(51) Int. Cl.: G01N 33/574

(54) **Diagnosing prostate cancer relapse**

(71) Applicant: IMG Institut für medizinische Genomforschung Planungsgesellschaft M.B.H., 1010 Vienna (AT)
(72) Inventor: Weinberger, Klaus, 6020, Innsbruck (AT); Deigner, Hans-Peter, 6020, Innsbruck (AT); Igwe, Emeka Ignatius, 6020, Innsbruck (AT); Enot, David, 14480 Creully (FR); Dallmann, Guido, 6020, Innsbruck (AT); Klocker, Helmut, 6020, Innsbruck (AT)
(74) Representative: Sonn & Partner Patentanwälte

(57) **Abstract**

The invention discloses the use of at least one substance selected from the group consisting of Phosphatidylcholine with diacyl residue sum C24:0 (PC aa C24:0); Phosphatidylcholine with diacyl residue sum C40:3 (PC ae C40:3); Phosphatidylcholine with diacyl residue sum C40:4 (PC ae C40:4); Lysophosphatidylcholine with acyl residue sum C26:0 (lysoPC a C26:0); Lysophosphatidylcholine with acyl residue sum C6:0 (lysoPC a C6:0); 13(S)-hydroxy-9Z,11E-octadecadienoic acid (13S-HODE); 12(S)-hydroxy-5Z,8Z,10E,14Z-eicosatetraenoic acid (12S-HETE); 15(S)-hydroxy-5Z,8Z,11Z,13E-eicosatetraenoic acid (15S-HETE); Leukotriene B4 (LTB4); Prostaglandin E2 (PGE2); Prostaglandin D2 (PGD2); 7α-Hydroxycholesterol (7aOHC); 7-Ketocholesterol (7KC); 5β,6β-Epoxycholesterol (5b,6b,EPC); 5α,6α-Epoxycholesterol (5a,6a,EPC); and 4β-Hydroxycholesterol (4BOHC); for prognosing relapse of a prostate cancer (PCa) in a sample of a body fluid or a tissue sample of a PCa patient.

## Description

The present invention relates to methods in diagnosing of prostate cancer patients.

Afflicting one out of nine men over age 65, prostate cancer (PCa) is a leading cause of male cancer-related death, second only to lung cancer. The American Cancer Society estimates that about 184,500 American men will be diagnosed with PCa and 39,200 will die in 2001.

Proper and early diagnosis of PCa is paramount for appropriate medical treatment to preserve the life of the patient. Currently, PCa is typically diagnosed with a digital rectal exam and/or prostate specific antigen (PSA) screening. An elevated serum PSA level can indicate the presence of PCa. PSA is used as a marker for PCa because it is secreted only by prostate cells. While healthy men have a low PSA serum it is raised in men with prostate cancer and further increased with the extent and the severity of the tumour. An elevated PSA level - especially when above 3 ng/ml - may raise a doctor's suspicion that a patient has PCa, while amounts above 50 in the majority of cancer cases indicate spread elsewhere in the body. Although PSA is considered as an effective tumour marker, it is not cancer specific. There is a considerable overlap in PSA concentrations in men with PCa and men with benign prostatic diseases such as prostatitis. Elevated levels of PSA are detectable in a large percentage of patients with non-malignant disorders, thus limiting the diagnostic specificity of serum PSA levels in PCa patients. Moreover, there is also variation in the levels of PSA in individuals from different races. Based on these findings, PSA serum levels alone are not exclusively used for the diagnosis of PCa. Recently, attempts have been made to improve the sensitivity of the PSA assay by relating the relationship of PSA levels to the size of the prostate (PSA density), or to measure the change of PSA levels over time (PSA velocity) or the ratio of bound to free PSA as an additional indication of the presence of clinically relevant PCa.

However, there is still need for more reliable diagnostic methods or tools for the identification and management of PCa. Nowadays, when PSA or digital rectal examination (DRE) indicates a strong likelihood that cancer is present, a transrectal ultrasound (TRUS) is used to map the prostate and show any suspicious areas. Biopsies of various sectors of the prostate are used to determine if PCa is present as well as determine the stage of the cancer.

In recent years, the advances in technologies in genomics and proteomics have led to the identification of several new candidate markers for PCa. Many new genes involved in PCa have been reported since the first microarray studies. Consequently, the long list of genes implicated in PCa includes Hepsin, a transmembrane serine protease, which is over-expressed in PCa compared to benign hyperplasia of normal prostate; RabGTPase-activating protein (PRC17), calcium-binding protein (S100-P), an actin polycomb group protein enhancer of zeste homologue 2 (EZH2), alpha-methylacyl-CoA racemase (AMACR), prostate-specific membrane antigen (PSMA), selenoprotein-P, Wnt signalling (Wnt 5A), Enlongin-C, androgen regulated gene (FKBP5, largely unknown), ANKH (regulates transportation of pyrophosphate, prostate A regulated transcript-1 (PART-1), prostate short chain DR1 (PSDR-1, Claudin-8, Gro2 and specific granule protein-28 (SG028). However, most of these investigations have concentrated on establishing unique associations of PCa with genetic or epigenetic determinants. In addition, linkage studies have a weak power to associate changes of small to moderate effects to disease outcomes.

Recently, metabolites, such as sarcosine, cysteine, glutamate, asparagine, glycine, leucine, proline, threonine, histidine, n-acetyl-aspartic acid (N-acetylaspartate (NAA)), inosine, inositol, adenosine, taurine, creatine, uric acid, glutathione, uracil, kynurenine, glycerol-s-phosphate, glycocholic acid, suberic acid, thymine, glutamic acid, xanthosine, 4-acetamidobutyric acid, citrate, malate, and N-acetylyrosine or thymine were identified in prostate tissue samples and/or in blood of PCa patients using mass spectrometry. In fact, the diagnostic value of biomarkers for PCa has been hampered by their poor sensitivity and specificity, and the non-conclusive understanding of the mechanisms controlling their differential levels in PCa. As a result of these limitations, the use of biomarkers is currently limited to research settings, and none has been recommended for routine assessment.

Early diagnosis of PCa is very important because this increases the chance of survival and the kind of the treatment, because treatment of PCa depends quite often on the stage of the cancer. Patients with a life expectancy of 10 years or less, whose Gleason score is low, and who have a localized tumour are often treated with watchful waiting (no treatment). Treatment options for more aggressive cancers include surgery (radical prostatectomy (RP)), chemotherapy and radiation application to kill or eliminate the cancer cells locally. Other methods of treating PCa include hormone therapy such as anti-androgen alone or in combination with surgery or radiation. Furthermore, luteinizing hormone-releasing hormones (LH-RH) analogs, which block the release of pituitary hormones stimulating testosterone production, have also been used for PCa treatment. However, the treatment of patients with hormones confines patients to hormonal injections for the rest of their lives, which may cause other side effects and could lead to hormone resistant tumours. For example, androgen ablation, is the most common therapy for advanced PCa, leading to massive apoptosis of androgen-dependent malignant cells and temporary tumour regression. In most cases, however, the tumour relapses and can proliferate independent of androgen supply. While surgical and radiation treatments are often effective for localized PCa, advanced disease remains incurable. Therefore, it is essential to have means for prognosing the risk or probability that a patient has with respect to such PCa relapses. Currently, the standard diagnostic means for detecting relapse of PCa is a steady measurement of PSA. Increases in postoperative serum PSA at levels of 0.001 to 0.1 µg/l after radical prostatectomy are associated with poor prognosis. Monitoring postoperative cases with a highly sensitive PSA assay (detection limit 0.001 µg/l) are suggested to detect clinically important biochemical relapse early after radical prostatectomy. These patients may be suitable for early intervention (e.g. radiation, hormone ablation therapy or chemotherapy).

Estimation of risk for relapse before RP is usually made on the basis of PSA (a higher PSA level indicates a higher risk for relapse) or tumour volume (a higher tumour volume (as well as tumour grade and tumour stage) directly correlates with the risk for relapse) (Cordon-Cardo et al., J.Clin. Invest. 117 (2007), 1876-1883). Other studies suggest time of diagnosis and PSA doubling time as major risk factors for PCa relapse - besides PSA values (Slovin et al., Clin. Cancer Res. 11 (2005), 8669-8673). In addition, it could also be shown that the surgeon's expertise correlates with the risk for PCa relapse.

Currently, however, PCa relapse is detected by PSA recurrence (concordance index being 0.77, sensitivity of 77% and specificity of 72% (Cordon-Cardo et al., 2007)).

Accordingly, there is still an urgent need for an early, rapid and reliable prognosis for PCa relapse which should preferably be present before RP, ideally requiring only minute amounts of samples which can easily be taken from a patient, such as blood. This is of great relevance for selection of proper type and time of treatment.

Therefore, the present invention provides the use of at least one substance selected from the group consisting of Phosphatidylcholine with diacyl residue sum C24:0 (PC aa C24:0); Phosphatidylcholine with diacyl residue sum C40:3 (PC ae C40:3); Phosphatidylcholine with diacyl residue sum C40:4 (PC ae C40:4); Lysophosphatidylcholine with acyl residue sum C26:0 (lysoPC a C26:0); Lysophosphatidylcholine with acyl residue sum C6:0 (lysoPC a C6:0); 13(S)-hydroxy-9Z,11E-octadecadienoic acid (13S-HODE); 12(S)-hydroxy-5Z,8Z,10E,14Z-eicosatetraenoic acid (12S-HETE); 15(S)-hydroxy-5Z,8Z,11Z,13E-eicosatetraenoic acid (15S-HETE); Leukotriene B4 (LTB4); Prostaglandin E2 (PGE2); Prostaglandin D2 (PGD2); 7α-Hydroxycholesterol (7aOHC); 7-Ketocholesterol (7KC); 5β,6β-Epoxycholesterol (5b,6b,EPC); 5α,6α-Epoxycholesterol (5a,6a,EPC); and 4β-Hydroxycholesterol (4BOHC); for prognosing the relapse of a prostate cancer (PCa) in a sample of a body fluid or a tissue sample of a PCa patient.

With the present invention, a superior set of small molecule biomarkers is provided, which allows a reliable prognosis for PCa relapse at an early stage, i.e. even before tumour surgery or tumour irradiation therapy. For example, the prognostic molecules according to the present invention have proven to allow a proper and statistically significant prognosis whether the tumour patient will show relapse within one, two or three years (or whether the patient will remain relapse-free for more than one, two or three years). The markers according to the present invention are molecules which are known to be involved in biochemical processes in the body. However, these molecules have never been discussed before as being relevant in connection with PCa relapse.

The determination of "fingerprints" of small molecules ("metabolomic profiles") is increasingly important in the field of diagnostics. The generation and the analysis of small molecule profiles of cells, cellular compartments, and specific organelles (e.g., mitochondria, Golgi, endoplasmic reticulum, cytoplasm, nucleus, etc.) is generally referred to as "metabolomics" ("the systematic study of the unique chemical fingerprints that specific cellular processes leave behind"; specifically, the study of their small-molecule metabolite profiles). Metabolites have the advantage that they directly reflect the biological processes in the cells, are easier to be profiled; and generally offer a new route for the identification of diagnostic targets.

Whereas such metabolomic profiles have already been investigated in PCa (Osl et al., Bioinform. 24 (2008), 2908-2914); Lokhov et al., Metabolomics DOI 10.1007/s11306-009-0187-x; Sreekumar et al., Nature 457 (2009), 910-914 (identifying sarcosine as one of the major differential metabolites distinguishing between tumour and non-tumour tissue)), nothing is known about metabolomics and differentiated metabolomics for PCa relapse risk.

Interestingly, the markers according to the present invention completely differ from the metabolites known to be indicative for PCa as indicated e.g. by Sreekumar et al.(2009). For example, sarcosine is definitely not a suitable marker for the present invention.

The marker set according to the present invention includes phosphaditylcholines (Phosphatidylcholine with diacyl residue sum C24:0 (PC aa C24:0); Phosphatidylcholine with diacyl residue sum C40:3 (PC ae C40:3); Phosphatidylcholine with diacyl residue sum C40:4 (PC ae C40:4)) lysophosphaditylcholines (Lysophosphatidylcholine with acyl residue sum C26:0 (lysoPC a C26:0); Lysophosphatidylcholine with acyl residue sum C6:0 (lysoPC a C6:0)) prostaglandins (13(S)-hydroxy-9Z,11E-octadecadienoic acid (13S-HODE); 12(S)-hydroxy-5Z,8Z,10E,14Z-eicosatetraenoic acid (12S-HETE); 15(S)-hydroxy-5Z,8Z,11Z,13E-eicosatetraenoic acid (15S-HETE); Leukotriene B4 (LTB4); Prostaglandin E2 (PGE2); Prostaglandin D2 (PGD2)), and oxysterols (7α-Hydroxycholesterol (7aOHC); 7-Ketocholesterol (7KC); 5β,6β-Epoxycholesterol (5b,6b,EPC); 5α,6α-Epoxycholesterol (5a,6a,EPC); and 4β-Hydroxycholesterol (4BOHC)). (Glycero-phospholipids are differentiated with respect to the presence of ester (a) and ether (e) bonds in the glycerol moiety, where two letters (aa, ea, or ee) denote that the first and the second position of the glycerol scaffold are bound to a fatty acid residue, whereas a single letter (a or e) indicates a bond with only one fatty acid residue; e.g. PC_aa_C24:0 denotes a phosphatidylcholine with 24 carbons in the two fatty acid side chains and no double bond in one of them).

Importantly, it has been shown with the present invention that other members of the compound classes phosphaditylcholines, lysophosphaditylcholines, prostaglandines and oxysterols have no marker function with respect to the PCa relapse status.

With the present invention it is possible to reliably predict whether a given patient has a certain risk for developing PCa relapse (i.e. to prognosticate whether he will develop PCa relapse or not). Knowing this prognosis, this risk can then be used for designing specific treatment of these patients (i.e. to decide on the optimal available treatment option and time of treatment) which could include more severe measures typically applied in PCa treatment (i.e. instead of "only RP" or "only irradiation" or "only hormone therapies", combinations of these measures or higher doses of medication/irradiation are applied). The results obtained by the present invention may also have implications for the RP itself which could be carried out in larger areas if the patient has a prognosis for PCa relapse. On the other hand, patients with no PCa relapse risk can be treated in a more convenient way.

Accordingly, the present invention also relates to a method for prognosing relapse of a prostate cancer (PCa) patient in a sample of a body fluid or a tissue sample of said PCa patient which is **characterised in that** at least one substance selected from the group consisting of Phosphatidylcholine with diacyl residue sum C24:0 (PC aa C24:0); Phosphatidylcholine with diacyl residue sum C40:3 (PC ae C40:3); Phosphatidylcholine with diacyl residue sum C40:4 (PC ae C40:4); Lysophosphatidylcholine with acyl residue sum C26:0 (lysoPC a C26:0); Lysophosphatidylcholine with acyl residue sum C6:0 (lysoPC a C6:0); 13(S)-hydroxy-9Z,11E-octadecadienoic acid (13S-HODE); 12(S)-hydroxy-5Z,8Z,10E,14Z-eicosatetraenoic acid (12S-HETE); 15(S)-hydroxy-5Z,8Z,11Z,13E-eicosatetraenoic acid (15S-HETE); Leukotriene B4 (LTB4); Prostaglandin E2 (PGE2); Prostaglandin D2 (PGD2); 7α-Hydroxycholesterol (7aOHC); 7-Ketocholesterol (7KC); 5β,6β-Epoxycholesterol (5b,6b,EPC); 5α,6α-Epoxycholesterol (5a,6a,EPC); and 4β-Hydroxycholesterol (4BOHC) is detected and quantified in said sample and the result of this quantification is compared with the amount of the at least one detected and quantified substance in a corresponding sample of known relapse status.

The samples for the present invention are derived from PCa tumour patients (which are, of course, always male human patients), especially patients that have already been confirmed to have PCa and expect RP. Preferably, the samples are taken and preferably also analysed according to the present invention before RP so that the results are available for the RP so that appropriate further measures can be foreseen even before or during surgery (e.g. appropriate planning of the kind and mode of surgery).

A specific advantage of the present invention is that the measurements can be carried out in blood or urine samples which can be obtained much easier than the prostate biopsy samples (or the excised prostate) which are usually investigated for full diagnosis of PCa patients. Therefore, the present method is preferably performed on a blood (or blood derived) sample (especially a blood, plasma or serum sample) or a urine sample. However, also tissue samples (e.g. prostate or liver tissue), cerebral spinal fluid samples, lymph samples, semen samples, etc. are also useable according to the present invention.

The results of the quantification of the substances according to the present invention are compared to the levels of reference for the substances tested, i.e. to levels which either prognose the development of PCa relapse or to levels which do not prognose the development of PCa relapse. Typically, reference levels for the metabolites according to the present invention are given, which are indicative for PCa relapse (or for having a reduced risk for PCa relapse). Preferably, the quantified levels for the samples according to the present invention are compared with such known reference levels. Therefore, in a preferred embodiment of the present invention, the amount of the at least one detected and quantified substance in a corresponding sample of known relapse status is a numerical limit for the amount of this substance.

The reference levels applied in the present method may also be tailored to specific techniques that are used to measure levels of metabolites in biological samples (e.g., LC-MS, GC-MS, etc.), where the levels of metabolites may differ based on the specific technique that is used. Establishment of such reference values for a given technique is easily possible for a person skilled in the art.

The sample analysis according to the present invention can be done with any method known and applied for metabolite fingerprinting. Usually, metabolomic analyses include the following important steps, which are well available to the person skilled in the art: First, the metabolites have to be extracted from the biological source (tissue sample or sample of a body fluid) in an efficient and unbiased manner. Specific care must be taken to prevent unwanted processes (such as oxidation) in the samples or at least take these processes into consideration when further processing and analysing those samples. Then the analytes can be separated, usually by chromatographical methods or electrophoresis, particularly capillary electrophoresis, liquid chromatography, especially HPLC, or gas chromatography. Detection, identification and quantification of the analytes are usually done by mass spectrometry (MS) or nuclear magnetic resonance (NMR) spectroscopy.

LC- or GC-MS is the most preferred way to develop metabolic profiles. In addition, mass spectral fingerprint libraries exist or can be developed that allow identification and (interlaboratorial) comparison of a metabolite according to its fragmentation pattern. MS is both sensitive and can be very specific. There are also a number of studies which use MS as a stand-alone technology: the sample is infused directly into the mass spectrometer with no prior separation, and the MS serves to both separate and to detect metabolites.

NMR does not rely on separation of the analytes, and the sample can thus be recovered for further analyses. All kinds of small molecule metabolites can be measured simultaneously - in this sense, NMR is close to being a universal detector. Practically, however, it is relatively insensitive compared to mass spectrometry-based techniques; additionally, NMR spectra can be very difficult to interpret for complex mixtures.

MS and NMR are by far the two leading technologies for metabolomics. Other methods of detection that have been used include electrochemical detection (coupled to HPLC), radiolabel (when combined with thin-layer chromatography), chemical modification assays (e.g. radioactive labeling), and enzymatic assays.

Preferably, the sample analysis with respect to the metabolites according to the present invention is performed by mass spectroscopy. Therefore, the method according to the present invention is preferably detected and quantified by mass spectroscopy, preferably liquid chromatography mass spectroscopy (LC-MS), especially high performance liquid chromatography mass spectroscopy (HPLC-MS) or reverse phase liquid chromatography mass spectroscopy (RPLC-MS); electro-spray ionisation mass spectroscopy (ESI-MS), gas chromatography mass spectroscopy (GC-MS), atmospheric pressure chemical ionisation mass spectroscopy (APCI-MS), capillary electrophoresis mass spectroscopy (CE-MS), tandem mass spectroscopy (MS-MS); or combinations of these mass spectroscopy techniques.

Each of the PCa marker substances according to the present invention is useable by itself (i.e. without the need for other markers) to establish the prognosis according to the present invention. Each of them shows statistically significant increase in pre-RP samples of patients with PCa relapses (except 12S-HETE, however, 12S-HETE shows performance as a marker by other selection approaches. A widely accepted threshold for statistical significance is generally "p value below 0.05". For omics applications, the "p value" is replaced by "q value" to take into account that the generation of many "p values" will increase the number of falsely significant results. A more robust approach consists in basing the selection on two or more statistical measures. In the present case, a metabolite is preferably entering the final selection if passing at least two criteria amongst: "q value below 0.1", "AUC greater than 0.7", "fold change greater than 20%". This is a preferred selection criterium for the present invention and additional modeling did come up with similar conclusions). However, due to the possibilities of mass spectrometers, it is very easy to analyse more than one marker in a sample. This allows an even more reliable and sensitive estimation for the PCa relapse prognosis. Accordingly, it is preferred that at least two, preferably at least three, especially at least four of the substances are determined, quantified and compared. Even more preferred, at least five, preferably at least ten, especially all sixteen, of the metabolites according to the present invention are analysed.

The prognosis developed according to the present invention may also further be combined with known prognosis factors for PCa relapse, such as pre-RP-PSA values, tumour size, grade and status, etc..

As a preferred reference value, amounts of the metabolites according to the present invention are compared with the values in a corresponding sample of a PCa relapse patient (i.e. a sample of known prognosis for PCa relapse). The "corresponding sample" is, of course, the sample that qualitatively corresponds to the sample measured according to the present invention, e.g. a blood sample corresponds to a reference blood sample or a urine sample corresponds to a reference urine sample. Preferably, the reference sample also corresponds to the measured sample concerning the detection method or even the detection apparatus (i.e. the same or the same type of mass spectrometer). Preferably, such a "real world" reference value is not only a single sample, but an average value based on a sample collective.

According to a preferred embodiment of the present invention, the amount of the at least one detected and quantified substance in a corresponding sample of known relapse status is therefore the amount of this substance in a sample of a PCa relapse patient or the average amount of this substance of a pool of samples of PCa relapse patients.

The prognosis markers according to the present invention are differentially upregulated in samples with expectation of PCa relapse.

This means that there is a statistically significant correlation between the increase of the substances (metabolites, PCa relapse markers) according to the present invention in samples of PCa patients and an increased risk of PCa relapse after RP. Otherwise, if these substances are not upregulated, the PCa patient has a good chance of not developing a relapse (e.g. in more than two or more than three years; there is even the chance that these patients do have significantly less relapse in PCa within a higher year number (e.g. at least five or at least ten years). Accordingly, in a preferred embodiment of the present method the sample is regarded as having a prognosis for PCa relapse, if the at least one substance is contained in an amount which is at least 10%, preferably at least 20%, more preferably at least 50 %, even more preferred at least 100%, especially at least 200%, increased compared to the amount of this substance in a sample of a PCa patient (or a group or even population of such patients in a given country, region or genetic pool) which does not have a prognosis for PCa relapse ("med FC"). Some of the marker substances provided according to the present invention have even significantly higher "-fold differences" (compared to the non PCa relapse). Any preferred combination of metabolite marker substances measures according to the present invention can be elaborated based on the information contained herein. Optimisation with respect to sensitivity, significance, specificity, etc. can be made by the person skilled in the art. In doing so, also the data contained herein, especially according to the disclosure in the example section, is specifically helpful. Preferably, the statistical methods mentioned and referred to in the example section are applied to the analysis according to the present invention, specifically for selecting reference values and statistical significance for attribution PCa relapse prognosis (or not having PCa relapse prognosis).

Preferred markers have at least two of the following properties: AUC of at least 0.70; med FC of at least 20, especially at least 50; and a p-value of 0.05 or lower, especially of 0.01 or lower.

When the amount(s) or level(s) of the one or more metabolites in the sample of the PCa patient is (are) determined, these amount(s) or level(s) may be compared to PCa relapse metabolite-reference levels, such as PCa relapse-positive and/or PCa relapse-negative reference levels to aid in diagnosing or to diagnose whether the subject has PCa relapse. To this end, it has also to be borne in mind that biological measurements always show natural variations which will be attended to by appropriate calibration of the individual systems. Objective values are therefore often problematic to establish whereas relative differences between the relapse/non relapse groups are easily providable for a given technique and/or apparatus set-up without the actual need for objective standard values being independent of the detection and quantification tools.

The method according to the present invention is in a preferred embodiment **characterised in that** the comparison is performed using software-based statistical and bioinformatic data analyses.

According to another aspect, the present invention relates to the use of a mass spectrometer for carrying out the present method by measuring one or more (or all) of the metabolites according to the present invention.

A further aspect of the present invention relates to a kit for carrying out the method according to the present invention. The kit according to the present invention comprises
- a mass spectrometer,
- a standard sample containing a known amount of at least one substance from the group of PCa relapse marker substances according to the present invention (and therefore a known status with respect to PCa relapse) and
- a sample of a PCa patient containing an unknown amount of at least one substance from the group of PCa relapse marker substances according to the present invention.

According to a preferred embodiment, the kit of the present invention is **characterised in that** it further comprises one or more of the following components: quality control samples, analyte standards, internal standards or data analysis software.

In some embodiments of the present invention, a computer-based analysis program is used to translate the raw data generated by the detection assay (e.g., the presence, absence, or amount of a PCa relapse marker metabolite) into data of predictive value for a clinician. The clinician can access the predictive data using any suitable means. Thus, in some embodiments, the present invention provides the further benefit that the clinician, who is not likely to be trained in metabolite analysis, need not understand the raw data. The data is presented directly to the clinician in its most useful form. The clinician is then able to immediately utilize the information in order to optimize the care of the subject.

The profile data is then prepared in a format suitable for interpretation by a treating clinician. For example, rather than providing raw data, the prepared format may represent a diagnosis or risk assessment (e.g., likelihood of PCa relapse being present) for the PCa patient, along with recommendations for particular treatment options. The data may be displayed to the clinician by any suitable method. For example, in some embodiments, the profiling service generates a report that can be printed for the clinician (e.g., at the point of care) or displayed to the clinician on a computer monitor.

The present invention is further described by the following examples, yet without being limited thereto.

### Materials and Methods

### Blood serum sample collection and processing

Serum samples were obtained from the PCa serum archive in Innsbruck, Austria. The serum sample procurement, data management and blood collection protocols were approved by the local Ethical Review Board. Blood samples from patients diagnosed with PCa were obtained from the Prostate Cancer Early Detection program open to the general public in Tyrol (Bartsch et al., BJU International 101 (2008), 809-816) with informed consent of sample donors. Blood samples were drawn by venous puncture using Sarstedt 9 ml z-gel serum monovettes, serum was obtained by centrifugation (4 min, 1800g) and frozen in 2ml cryovials (Simport) at -80°C. At first use the sera were distributed into aliquots to minimize freeze-thaw cycles.

### Patient and control cohorts

Serum samples were obtained prior to treatment from PCa patients who underwent radical prostatectomy after cancer diagnosis. The inclusion criteria for PCa patients required the histopathological assessment of radical prostatectomy specimens. Tumour patients with progression after radical prostatectomy were defined as patients in follow-up control with rising PSA serum levels (increase in at least two consecutive measurements from 0 to above 0,2 ng/ml) or patients who's PSA nadir (i.e. the lowest PSA reading achieved after any treatment for PC; a PSA nadir of 0.1 ng/ml or lower indicates undetectable PSA) was >0 after radical prostatectomy and increased thereafter. Clinical data of the patients were retrieved from the clinical databases as well as patients' health records.

### General Analytics:

Sample preparation and metabolomic analyses were performed at BIOCRATES life sciences AG, Innsbruck, Austria. A multiparametric, highly robust, sensitive and high-throughput targeted metabolomic platform was used consisting of flow injection analysis (FIA)-MS/MS and LC-MS/MS methods for the simultaneous quantification of a broad range of endogenous intermediates namely from the panel disclosed in table 1. All procedures (sample handling, analytics) were performed by co-workers blinded to the groups.

### Plasma homogenization

Plasma samples were prepared by standard procedures and stored at (-70°C). To enable analysis of all samples simultaneously within one batch, samples were thawed on ice (1 h) on the day of analysis and centrifuged at 18000 g at 2°C for 5 min. All tubes were prepared with 0.001% BHT (butylated hydroxytoluene; Sigma-Aldrich, Vienna, Austria) to prevent artificial formation of prostaglandins caused by autooxidation.

### Acylcarnitines, Sphingomyelins, Hexoses, Glycerophospholipids (FIA-MS/MS)

To determine the concentration of acylcarnitines, sphingomyelins and glycerophospholipids in brain homogenates and in plasma, the AbsoluteIDQ kit p150 (Biocrates Life Sciences AG) was prepared as described in the manufacturer's protocol. In brief, 10 µL of brain homogenate was added to the center of the filter on the upper 96-well kit plate, and the samples were dried using a nitrogen evaporator (VLM Laboratories). Subsequently, 20 µL of a 5 % solution of phenyl-isothiocyanate was added for derivatization. After incubation, the filter spots were dried again using an evaporator. The metabolites were extracted using 300 µL of a 5 mM ammonium acetate solution in methanol. The extracts were obtained by centrifugation into the lower 96-deep well plate followed by a dilution step with 600 µL of kit MS running solvent. Mass spectrometric analysis was performed on an API4000 QTrap® tandem mass spectrometry instrument (Applied Biosystems/MDS Analytical Technologies) equipped with an electro-spray ionization (ESI)-source using the analysis acquisition method as provided in the AbsoluteIDQ kit. The standard FIA-MS/MS method was applied for all measurements with two subsequent 20 µL injections (one for positive and one for negative mode analysis). Multiple reaction monitoring (MRM) detection was used for quantification applying the spectra parsing algorithm integrated into the MetIQ software (Biocrates Life Sciences AG). Concentration values for 148 metabolites (all analytes determined with the metabolomics kit besides of the amino acids, which were determined by a different method) obtained by internal calibration were exported for comprehensive statistical analysis.

### Amino acids, Biogenic amines (LC-MS/MS)

Amino acids and biogenic amines were quantitatively analysed by reversed phase LC-MS/MS to obtain chromatographic separation of isobaric (same MRM ion pairs) metabolites for individual quantitation performed by external calibration and by use of internal standards. 10 µL sample volume (plasma, brain homogenate) is required for the analysis using the following sample preparation procedure. Samples were added on filter spots placed in a 96-solvinert well plate (internal standards were placed and dried down under nitrogen before), fixed above a 96 deep well plate (capture plate). 20 µL of 5% phenyl-isothiocyanate derivatization reagent was added. The derivatized samples were extracted after incubation by aqueous methanol into the capture plate. Sample extracts were analyzed by LC-ESI-MS/MS in positive MRM detection mode with an API4000 QTrap® tandem mass spectrometry instrument (Applied Biosystems/MDS Analytical Technologies). The analyzed individual metabolite concentrations (Analyst 1.4.2 software, Applied Biosystems) were exported for comprehensive statistical analysis.

### Bile acids (LC-MS/MS)

A highly selective reversed phase LC-MS/MS analysis method in negative MRM detection mode was applied to determine the concentration of bile acids in plasma samples. Samples were extracted via dried filter spot technique in 96 well plate format, which is well suitable for high throughput analysis. For highly accurate quantitation internal standards and external calibration were applied. In brief, internal standards and 20 µL sample volume placed onto filter spots were extracted and simultaneously protein precipitated with aqueous methanol. These sample extracts were measured by LC-ESI-MS/MS with an API4000 QTrap® tandem mass spectrometry instrument (Applied Biosystems/MDS Analytical Technologies). Data of bile acids were quantified with Analyst 1.4.2 software (Applied Biosystems) and finally exported for comprehensive statistical analysis.

### Prostanoids, oxidised fatty acids (LC-MS/MS)

Prostanoids - a term summarizing prostaglandins (PG), thromboxanes (TX) and prostacylines - and oxidised fatty acid metabolites were analyzed in plasma extracts by LC-ESI-MS/MS (Unterwurzacher et al. Clin Chem Lab Med 46 (11) (2008), 1589-1597) and in brain homogenate extracts by online solid phase extraction (SPE)-LC-MS/MS with an API4000 QTrap® tandem mass spectrometry instrument (Applied Biosystems/MDS Analytical Technologies) in negative MRM detection mode. The sample preparation was the same for both, plasma and brain homogenates. In brief, filter spots in a 96 well plate were spiked with internal standard; 20 µL of plasma or tissue homogenates were added and extracted with aqueous methanol, the individual extracts then were analysed. Data of prostanoids and oxidised fatty acids were quantified with Analyst 1.4.2 software (Applied Biosystems) and finally exported for statistical analysis.

### Oxysterols

Oxysterols are determined after extraction and saponification by HPLC-Tandem mass spectrometer (HPLC-API-MS/MS) in positive detection mode using Multiple Reaction Mode (MRM).

Samples (20 µL), calibrators and internal standard mixture were placed into a capture plate and were protein precipitated in the first step by means of addition of 200 µL acetonitrile and centrifugation. 180 µL of the appropriate supernatants were transferred on a new filter plate with 7 mm filter spots, dried down, hydrolysed with 0.35 M KOH in 95 % Ethanol and after washing steps extracted with 100 µL aqueous MeOH. An aliquot of the extracted sample is injected onto the HPLC-MS/MS system. Chromatographic separation and detection is performed by using a Zorbax Eclipse XDB C18, 150 x 2.0 mm, 3.5 µm HPLC-Column at a flow rate of 0.3 mL/min followed by electrospray ionization on the API4000/QTRAP4000 tandem mass spectrometer. For the quantitation the Analyst Quantitation software from Applied Bioystems was used.

### Energy metabolism (Organic Acids) (LC-MS/MS)

For the quantitative analysis of energy metabolism intermediates (glycolysis, citrate cycle, pentose phosphate pathway, urea cycle) hdyrophilic interaction liquid chromatography (HILIC)-ESI-MS/MS method in highly selective negative MRM detection mode was used. The MRM detection was performed using an API4000 QTrap® tandem mass spectrometry instrument (Applied Biosystems/MDS Analytical Technologies). 20 µL sample volume (plasma, brain homogenate) was protein precipitated and extracted simultaneously with aqueous methanol in a 96 well plate format. Internal standards (ratio external to internal standard) and external calibration were used for highly accurate quantitation. Data were quantified with Analyst 1.4.2 software (Applied Biosystems) and finally exported for statistical analysis.

**Table 1: investigated metabolites**

| Name | Family | Common me |
|---|---|---|
| C0 | Ac.Ca. | Carnitine (free) |
| C10 | Ac.Ca. | Decanoylcarnitine [Caprylcarnitine] (Fumarylcarnitine) |
| C10:1 | Ac.Ca. | Decenoylcarnitine |
| C10:2 | Ac.Ca. | Decadienoylcarnitine |
| C12 | Ac.Ca. | Dodecanoylcarnitine [Laurylcarnitine] |
| C12-DC | Ac.Ca. | Dodecanedioylcarnitine |
| C12:1 | Ac.Ca. | Dodecenoylcarnitine |
| C14 | Ac.Ca. | Tetradecanoylcarnitine |
| C14:1 | Ac.Ca. | Tetradecenoylcarnitine [Myristoleylcarnitine] |
| C14:1-OH | Ac.Ca. | 3-Hydroxytetradecenoylcarnitine [3-Hydroxymyristoleylcarnitine] |
| C14:2 | Ac.Ca. | Tetradecadienoylcarnitine |
| C14:2-OH | Ac.Ca. | 3-Hydroxytetradecadienoylcarnitine |
| C16 | Ac.Ca. | Hexadecanoylcarnitine [Palmitoylcarnitine] |
| C16-OH | Ac.Ca. | 3-Hydroxyhexadecanolycarnitine [3-Hydroxypalmitoylcarnitine] |
| C16:1 | Ac.Ca. | Hexadecenoylcarnitine [Palmitoleylcarnitine] |
| C16:1-OH | Ac.Ca. | 3-Hydroxyhexadecenoylcarnitine [3-Hydroxypalmitoleylcarnitine] |
| C16:2 | Ac.Ca. | Hexadecadienoylcarnitine |
| C16:2-OH | Ac.Ca. | 3-Hydroxyhexadecadienoylcarnitine |
| C18 | Ac.Ca. | Octadecanoylcarnitine [Stearylcarnitine] |
| C18:1 | Ac.Ca. | Octadecenoylcarnitine [Oleylcarnitine] |
| C18:1-OH | Ac.Ca. | 3-Hydroxyoctadecenoylcarnitine [3-Hydroxyoleylcarnitine] |
| C18:2 | Ac.Ca. | Octadecadienoylcarnitine [Linoleylcarnitine] |
| C2 | Ac.Ca. | Acetylcarnitine |
| C3 | Ac.Ca. | Propionylcarnitine |
| C3-OH | Ac.Ca. | Hydroxypropionylcarnitine |
| C3:1 | Ac.Ca. | Propenoylcarnitine |
| C4 | Ac.Ca. | Butyrylcarnitine / Isobutyrylcarnitine |
| C4-OH (C3-DC) | Ac.Ca. | 3-Hydroxybutyrylcarnitine / Malonylcarnitine |
| C4:1 | Ac.Ca. | Butenoylcarnitine |
| C5 | Ac.Ca. | Isovalerylcarnitine / 2-Methylbutyrylcarnitine / Valerylcarmtme |
| C5-DC (C6-OH) | Ac.Ca. | Glutarylcarnitine / Hydroxycaproylcarnitine |
| C5-M-DC | Ac.Ca. | Methylglutarylcarnitine |
| C5-OH(C3-DC-M) | Ac.Ca. | 3-Hydroxyisovalerylcarnitine / 3-Hydroxy-2-methylbutyryl |
| C5:1 | Ac.Ca. | Tiglylcarnitine / 3-Methyl-crotonylcarnitine |
| C5:1-DC | Ac.Ca. | Tiglylcarnitine / 3-Methyl-crotonylcarnitine |
| C6 (C4:1-DC) | Ac.Ca. | Hexanoylcarnitine [Caproylcarnitine] |
| C6:1 | Ac.Ca. | Hexenoylcarnitine |
| C7-DC | Ac.Ca. | Pimelylcarnitine |
| C8 | Ac.Ca. | Octanoylcarnitine [Caprylylcarnitine] |
| C8:1 | Ac.Ca. | Octenoylcarnitine |
| C9 | Ac.Ca. | Nonoylcarnitine [Pelargonylcarnitine] |
| SM (OH) C14:1 | S.L. | Sphingomyelin with acyl residue sum (OH) C14:1 |
| SM (OH) C16:1 | S.L. | Sphingomyelin with acyl residue sum (OH) C16:1 |
| SM (OH) C22:1 | S.L. | Sphingomyelin with acyl residue sum (OH) C22:1 |
| SM (OH) C22:2 | S.L. | Sphingomyelin with acyl residue sum (OH) C22:2 |
| SM (OH) C24:1 | S.L. | Sphingomyelin with acyl residue sum (OH) C24:1 |
| SM C26:0 | S.L. | Sphingomyelin with acyl residue sum C26:0 |
| SM C26:1 | S.L. | Sphingomyelin with acyl residue sum C26:1 |
| PC aa C24:0 | GP.L. | Phosphatidylcholine with diacyl residue sum C24:0 |
| PC aa C26:0 | GP.L. | Phosphatidylcholine with diacyl residue sum C26:0 |
| PC aa C28:1 | GP.L. | Phosphatidylcholine with diacyl residue sum C28:1 |
| PC aa C32:3 | GP.L. | Phosphatidylcholine with diacyl residue sum C32:3 |
| PC aa C34:4 | GP.L. | Phosphatidylcholine with diacyl residue sum C34:4 |
| PC aa C36:6 | GP.L. | Phosphatidylcholine with diacyl residue sum C36:6 |
| PC aa C38:0 | GP.L. | Phosphatidylcholine with diacyl residue sum C38:0 |
| PC aa C40:1 | GP.L. | Phosphatidylcholine with diacyl residue sum C40:1 |
| PC aa C40:2 | GP.L. | Phosphatidylcholine with diacyl residue sum C40:2 |
| PC aa C40:3 | GP.L. | Phosphatidylcholine with diacyl residue sum C40:3 |
| PC aa C42:0 | GP.L. | Phosphatidylcholine with diacyl residue sum C42:0 |
| PC aa C42:1 | GP.L. | Phosphatidylcholine with diacyl residue sum C42:1 |
| PC aa C42:2 | GP.L. | Phosphatidylcholine with diacyl residue sum C42:2 |
| PC aa C42:4 | GP.L. | Phosphatidylcholine with diacyl residue sum C42:4 |
| PC aa C42:5 | GP.L. | Phosphatidylcholine with diacyl residue sum C42:5 |
| PC aa C42:6 | GP.L. | Phosphatidylcholine with diacyl residue sum C42:6 |
| PC ae C30:0 | GP.L. | Phosphatidylcholine with acyl-alkyl residue sum C30:0 |
| PC ae C30:1 | GP.L. | Phosphatidylcholine with acyl-alkyl residue sum C30:1 |
| PC ae C30:2 | GP.L. | Phosphatidylcholine with acyl-alkyl residue sum C30:2 |
| PC ae C32:2 | GP.L. | Phosphatidylcholine with acyl-alkyl residue sum C32:2 |
| PC ae C36:0 | GP.L. | Phosphatidylcholine with acyl-alkyl residue sum C36:0 |
| PC ae C38:0 | GP.L. | Phosphatidylcholine with acyl-alkyl residue sum C38:0 |
| PC ae C40:0 | GP.L. | Phosphatidylcholine with acyl-alkyl residue sum C40:0 |
| PC ae C40:1 | GP.L. | Phosphatidylcholine with acyl-alkyl residue sum C40:1 |
| PC ae C40:2 | GP.L. | Phosphatidylcholine with acyl-alkyl residue sum C40:2 |
| PC ae C40:3 | GP.L. | Phosphatidylcholine with acyl-alkyl residue sum C40:3 |
| PC ae C40:4 | GP.L. | Phosphatidylcholine with acyl-alkyl residue sum C40:4 |
| PC ae C40:6 | GP.L. | Phosphatidylcholine with acyl-alkyl residue sum C40:6 |
| PC ae C42:0 | GP.L. | Phosphatidylcholine with acyl-alkyl residue sum C42:0 |
| PC ae C42:1 | GP.L. | Phosphatidylcholine with acyl-alkyl residue sum C42:1 |
| PC ae C42:2 | GP.L. | Phosphatidylcholine with acyl-alkyl residue sum C42:2 |
| PC ae C42:3 | GP.L. | Phosphatidylcholine with acyl-alkyl residue sum C42:3 |
| PC ae C42:4 | GP.L. | Phosphatidylcholine with acyl-alkyl residue sum C42:4 |
| PC ae C42:5 | GP.L. | Phosphatidylcholine with acyl-alkyl residue sum C42:5 |
| PC ae C44:3 | GP.L. | Phosphatidylcholine with acyl-alkyl residue sum C44:3 |
| PC ae C44:4 | GP.L. | Phosphatidylcholine with acyl-alkyl residue sum C44:4 |
| PC ae C44:5 | GP.L. | Phosphatidylcholine with acyl-alkyl residue sum C44:5 |
| PC ae C44:6 | GP.L. | Phosphatidylcholine with acyl-alkyl residue sum C44:6 |
| lysoPC a C14:0 | GP.L. | Lysophosphatidylcholine with acyl residue sum C14:0 |
| lysoPC a C16:1 | GP.L. | Lysophosphatidylcholine with acyl residue sum C16:1 |
| lysoPC a C17:0 | GP.L. | Lysophosphatidylcholine with acyl residue sum C17:0 |
| lysoPC a C20:3 | GP.L. | Lysophosphatidylcholine with acyl residue sum C20:3 |
| lysoPC a C24:0 | GP.L. | Lysophosphatidylcholine with acyl residue sum C24:0 |
| lysoPC a C26:0 | GP.L. | Lysophosphatidylcholine with acyl residue sum C26:0 |
| lysoPC a C26:1 | GP.L. | Lysophosphatidylcholine with acyl residue sum C26:1 |
| lysoPC a C28:0 | GP.L. | Lysophosphatidylcholine with acyl residue sum C28:0 |
| lysoPC a C28:1 | GP.L. | Lysophosphatidylcholine with acyl residue sum C28:1 |
| lysoPC a C6:0 | GP.L. | Lysophosphatidylcholine with acyl residue sum C6:0 |
| Gly | Am.Ac. | Glycine |
| Ala | Am.Ac. | Alanine |
| Ser | Am.Ac. | Serine |
| Pro | Am.Ac. | Proline |
| Val | Am.Ac. | Valise |
| Thr | Am.Ac. | Threonine |
| Xle | Am.Ac. | Leucine + Isoleucine |
| Leu | Am.Ac. | Leucine |
| Ile | Am.Ac. | Isoleucine |
| Asn | Am.Ac. | Asparagine |
| Asp | Am.Ac. | Aspartate |
| Gin | Am.Ac. | Glutamine |
| Glu | Am.Ac. | Glutamate |
| Met | Am.Ac. | Methionine |
| His | Am.Ac. | Histidine |
| Phe | Am.Ac. | Phenylalanine |
| Arg | Am.Ac. | Arginine |
| Cit | Am.Ac. | Citrulline |
| Tyr | Am.Ac. | Tyrosine |
| Trp | Am.Ac. | Tryptophan |
| Orn | Am.Ac. | Ornithine |
| Lys | Am.Ac. | Lysine |
| ADMA | B.Am. | asymmetrical Dimethylarginin |
| totalDMA | B.Am. | Total dimethylarginine: sum ADMA + SDMA |
| Met-SO | Am.Ac. | Methionine-Sulfoxide |
| Kynurenine | B.Am. | Kynurenine |
| Putrescine | B.Am. | Putrescine |
| Spermidine | B.Am. | Spermidine |
| Spermine | B.Am. | Spermine |
| Creatinine | B.Am. | Creatinine |
| 13S-HODE | P.G. | 13(S)-hydroxy-9Z,11E-octadecadienoic acid |
| 12S-HETE | P.G. | 12(S)-hydroxy-5Z,8Z,10E,14Z-elcosatetraenoic acid |
| 15S-HETE | P.G. | 15(S)-hydroxy-5Z,8Z,11Z,13E-elcosatetraenoic acid |
| LTB4 | P.G. | Leukotriene B4 |
| DHA | P.G. | Docosahexaenoic acid |
| PGE2 | P.G. | Prostaglandin E2 |
| PGD2 | P.G. | Prostaglandin D2 |
| AA | P.G. | Arachidonic acid |
| Lac | En.Met. | Lactate |
| Suc | En.Met. | Succinic acid (succite) |
| Hex | En.Met. | Hexose pool |
| 22ROHC | O.St. | 22-R-Hydroxycholesterol |
| 24SOHC | O.St. | 24-S-Hydroxycholesterol |
| 250HC | O.St. | 25-Hydroxycholesterol |
| 270HC | O.St. | 27-Hydroxycholesterol |
| 3B,5a,6BTHC | O.St. | 3β,5α,6β-Trihydroxycholestan |
| 7aOHC | O.St. | 7α-Hydroxycholesterol |
| 7KC | O.St. | 7-Ketocholesterol |
| 5a,6a,EPC | O.St. | 5α,6α-Epoxycholesterol |
| 4BOHC | O.St. | 4β-Hydroxycholesterol |
| Desmosterol | O.St. | Desmosterol |
| 7DHC | O.St. | 7-Dehydrocholesterol (Vitamin D3) |
| Lanosterol | O.St. | Lanosterol |
| 20aOHC | O.St. | 20α-Hydroxycholesterol |
| 22SOHC | O.St. | 22S-Hydroxycholesterol |
| 24,25EC | O.St. | 24,25-Epoxycholesterol |
| 5B,6B,EC | O.St. | 5B,6B-Epoxycholesterol |
| 5a,6a,EC | O.St. | 5α,6α-Epoxycholesterol |
| Cholestenone | O.St. | Cholestenone |
| PE a C16:0 | GP.L. | Lysophosphatidylethanolamine with acyl residue sum C16:0 |
| PE a C18:0 | GP.L. | Lysophosphatidylethanolamine with acyl residue sum C18:0 |
| PE a C18:1 | GP.L. | Lysophosphatidylethanolamine with acyl residue sum C18:1 |
| PE a C18:2 | GP.L. | Lysophosphatidylethanolamine with acyl residue sum C18:2 |
| PE a C20:4 | GP.L. | Lysophosphatidylethanolamine with acyl residue sum C20:4 |
| PE a C22:4 | GP.L. | Lysophosphatidylethanolamine with acyl residue sum C22:4 |
| PE a C22:5 | GP.L. | Lysophosphatidylethanolamine with acyl residue sum C22:5 |
| PE a C22:6 | GP.L. | Lysophosphatidylethanolamine with acyl residue sum C22:6 |
| PE e C18:0 | GP.L. | Lysophosphatidylethanolamine with alkyl residue sum C18:0 |
| PG e C14:2 | GP.L. | Lysophosphatidylglycerol with alkyl residue sum C14:2 |
| PE aa C20:0 | GP.L. | Phosphatidylethanolamine with diacyl residue sum C20:0 |
| PE aa C22:2 | GP.L. | Phosphatidylethanolamine with diacyl residue sum C22:2 |
| PE aa C26:4 | GP.L. | Phosphatidylethanolamine with diacyl residue sum C26:4 |
| PE aa C28:4 | GP.L. | Phosphatidylethanolamine with diacyl residue sum C28:4 |
| PE aa C28:5 | GP.L. | Phosphatidylethanolamine with diacyl residue sum C28:5 |
| PE aa C34:0 | GP.L. | Phosphatidylethanolamine with diacyl residue sum C34:0 |
| PE aa C34:1 | GP.L. | Phosphatidylethanolamine with diacyl residue sum C34:1 |
| PE aa C34:2 | GP.L. | Phosphatidylethanolamine with diacyl residue sum C34:2 |
| PE aa C34:3 | GP.L. | Phosphatidylethanolamine with diacyl residue sum C34:3 |
| PE aa C36:0 | GP.L. | Phosphatidylethanolamine with diacyl residue sum C36:0 |
| PE aa C36:1 | GP.L. | Phosphatidylethanolamine with diacyl residue sum C36:1 |
| PE aa C36:2 | GP.L. | Phosphatidylethanolamine with diacyl residue sum C36:2 |
| PE aa C36:3 | GP.L. | Phosphatidylethanolamine with diacyl residue sum C36:3 |
| PE aa C36:4 | GP.L. | Phosphatidylethanolamine with diacyl residue sum C36:4 |
| PE aa C36:5 | GP.L. | Phosphatidylethanolamine with diacyl residue sum C36:5 |
| PE aa C38:0 | GP.L. | Phosphatidylethanolamine with diacyl residue sum C38:0 |
| PE aa C38:1 | GP.L. | Phosphatidylethanolamine with diacyl residue sum C38:1 |
| PE aa C38:2 | GP.L. | Phosphatidylethanolamine with diacyl residue sum C38:2 |
| PE aa C38:3 | GP.L. | Phosphatidylethanolamine with diacyl residue sum C38:3 |
| PE aa C38:4 | GP.L. | Phosphatidylethanolamine with diacyl residue sum C38:4 |
| PE aa C38:5 | GP.L. | Phosphatidylethanolamine with diacyl residue sum C38:5 |
| PE aa C38:6 | GP.L. | Phosphatidylethanolamine with diacyl residue sum C38:6 |
| PE aa C38:7 | GP.L. | Phosphatidylethanolamine with diacyl residue sum C38:7 |
| PE aa C40:2 | GP.L. | Phosphatidylethanolamine with diacyl residue sum C40:2 |
| PE aa C40:3 | GP.L. | Phosphatidylethanolamine with diacyl residue sum C40:3 |
| PE aa C40:4 | GP.L. | Phosphatidylethanolamine with diacyl residue sum C40:4 |
| PE aa C40:5 | GP.L. | Phosphatidylethanolamine with diacyl residue sum C40:5 |
| PE aa C40:6 | GP.L. | Phosphatidylethanolamine with diacyl residue sum C40:6 |
| PE aa C40:7 | GP.L. | Phosphatidylethanolamine with diacyl residue sum C40:7 |
| PE aa C48:1 | GP.L. | Phosphatidylethanolamine with diacyl residue sum C48:1 |
| PE ae C34:1 | GP.L. | Phosphatidylethanolamine with acyl-alkyl residue sum C34:1 |
| PE ae C34:2 | GP.L. | Phosphatidylethanolamine with acyl-alkyl residue sum C34:2 |
| PE ae C34:3 | GP.L. | Phosphatidylethanolamine with acyl-alkyl residue sum C34:3 |
| PE ae C36:1 | GP.L. | Phosphatidylethanolamine with acyl-alkyl residue sum C36:1 |
| PE ae C36:2 | GP.L. | Phosphatidylethanolamine with acyl-alkyl residue sum C36:2 |
| PE ae C36:3 | GP.L. | Phosphatidylethanolamine with acyl-alkyl residue sum C36:3 |
| PE ae C36:4 | GP.L. | Phosphatidylethanolamine with acyl-alkyl residue sum C36:4 |
| PE ae C36:5 | GP.L. | Phosphatidylethanolamine with acyl-alkyl residue sum C36:5 |
| PE ae C38:1 | GP.L. | Phosphatidylethanolamine with acyl-alkyl residue sum C38:1 |
| PE ae C38:2 | GP.L. | Phosphatidylethanolamine with acyl-alkyl residue sum C38:2 |
| PE ae C38:3 | GP.L. | Phosphatidylethanolamine with acyl-alkyl residue sum C38:3 |
| PE ae C38:4 | GP.L. | Phosphatidylethanolamine with acyl-alkyl residue sum C38:4 |
| PE ae C38:5 | GP.L. | Phosphatidylethanolamine with acyl-alkyl residue sum C38:5 |
| PE ae C38:6 | GP.L. | Phosphatidylethanolamine with acyl-alkyl residue sum C38:6 |
| PE ae C40:1 | GP.L. | Phosphatidylethanolamine with acyl-alkyl residue sum C40:1 |
| PE ae C40:2 | GP.L. | Phosphatidylethanolamine with acyl-alkyl residue sum C40:2 |
| PE ae C40:3 | GP.L. | Phosphatidylethanolamine with acyl-alkyl residue sum C40:3 |
| PE ae C40:4 | GP.L. | Phosphatidylethanolamine with acyl-alkyl residue sum C40:4 |
| PE ae C40:5 | GP.L. | Phosphatidylethanolamine with acyl-alkyl residue sum C40:5 |
| PE ae C40:6 | GP.L. | Phosphatidylethanolamine with acyl-alkyl residue sum C40:6 |
| PE ae C42:1 | GP.L. | Phosphatidylethanolamine with acyl-alkyl residue sum C42:1 |
| PE ae C42:2 | GP.L. | Phosphatidylethanolamine with acyl-alkyl residue sum C42:2 |
| PE ae C46:5 | GP.L. | Phosphatidylethanolamine with acyl-alkyl residue sum C46:5 |
| PE ae C46:6 | GP.L. | Phosphatidylethanolamine with acyl-alkyl residue sum C46:6 |
| PG aa C30:0 | GP.L. | Phosphatidylglycerol with diacyl residue sum C30:0 |
| PG aa C32:0 | GP.L. | Phosphatidylglycerol with diacyl residue sum C32:0 |
| PG aa C32:1 | GP.L. | Phosphatidylglycerol with diacyl residue sum C32:1 |
| PG aa C33:6? | GP.L. | Phosphatidylglycerol with diacyl residue sum C33:6? |
| PG aa C34:0 | GP.L. | Phosphatidylglycerol with diacyl residue sum C34:0 |
| PG aa C34:1 | GP.L. | Phosphatidylglycerol with diacyl residue sum C34:1 |
| PG aa C34:2 | GP.L. | Phosphatidylglycerol with diacyl residue sum C34:2 |
| PG aa C34:3 | GP.L. | Phosphatidylglycerol with diacyl residue sum C34:3 |
| PG aa C36:0 | GP.L. | Phosphatidylglycerol with diacyl residue sum C36:0 |
| PG aa C36:1 | GP.L. | Phosphatidylglycerol with diacyl residue sum C36:1 |
| PG aa C36:2 | GP.L. | Phosphatidylglycerol with diacyl residue sum C36:2 |
| PG aa C36:3 | GP.L. | Phosphatidylglycerol with diacyl residue sum C36:3 |
| PG aa C36:4 | GP.L. | Phosphatidylglycerol with diacyl residue sum C36:4 |
| PG aa C38:5 | GP.L. | Phosphatidylglycerol with diacyl residue sum C38:5 |
| PG ae C32:0 | GP.L. | Phosphatidylglycerol with acyl-alkyl residue sum C32:0 |
| PG ae C34:0 | GP.L. | Phosphatidylglycerol with acyl-alkyl residue sum C34:0 |
| PG ae C34:1 | GP.L. | Phosphatidylglycerol with acyl-alkyl residue sum C34:1 |
| PG ae C36:1 | GP.L. | Phosphatidylglycerol with acyl-alkyl residue sum C36:1 |
| PS aa C34:1 | GP.L. | Phosphatidylserine with diacyl residue sum C34:1 |
| PS aa C34:2 | GP.L. | Phosphatidylserine with diacyl residue sum C34:2 |
| PS aa C36:0 | GP.L. | Phosphatidylserine with diacyl residue sum C36:0 |
| PS aa C36:1 | GP.L. | Phosphatidylserine with diacyl residue sum C36:1 |
| PS aa C36:2 | GP.L. | Phosphatidylserine with diacyl residue sum C36:2 |
| PS aa C36:3 | GP.L. | Phosphatidylserine with diacyl residue sum C36:3 |
| PS aa C36:4 | GP.L. | Phosphatidylserine with diacyl residue sum C36:4 |
| PS aa C38:1 | GP.L. | Phosphatidylserine with diacyl residue sum C38:1 |
| PS aa C38:2 | GP.L. | Phosphatidylserine with diacyl residue sum C38:2 |
| PS aa C38:3 | GP.L. | Phosphatidylserine with diacyl residue sum C38:3 |
| PS aa C38:4 | GP.L. | Phosphatidylserine with diacyl residue sum C38:4 |
| PS aa C38:5 | GP.L. | Phosphatidylserine with diacyl residue sum C38:5 |
| PS aa C40:1 | GP.L. | Phosphatidylserine with diacyl residue sum C40:1 |
| PS aa C40:2 | GP.L. | Phosphatidylserine with diacyl residue sum C40:2 |
| PS aa C40:3 | GP.L. | Phosphatidylserine with diacyl residue sum C40:3 |
| PS aa C40:4 | GP.L. | Phosphatidylserine with diacyl residue sum C40:4 |
| PS aa C40:5 | GP.L. | Phosphatidylserine with diacyl residue sum C40:5 |
| PS aa C40:6 | GP.L. | Phosphatidylserine with diacyl residue sum C40:6 |
| PS aa C40:7 | GP.L. | Phosphatidylserine with diacyl residue sum C40:7 |
| PS aa C42:1 | GP.L. | Phosphatidylserine with diacyl residue sum C42:1 |
| PS aa C42:2 | GP.L. | Phosphatidylserine with diacyl residue sum C42:2 |
| PS aa C42:4 | GP.L. | Phosphatidylserine with diacyl residue sum C42:4 |
| PS aa C42:5 | GP.L. | Phosphatidylserine with diacyl residue sum C42:5 |
| PS ae C34:2 | GP.L. | Phosphatidylserine with acyl-alkyl residue sum C34:2 |
| PS ae C36:1 | GP.L. | Phosphatidylserine with acyl-alkyl residue sum C36:1 |
| PS ae C36:2 | GP.L. | Phosphatidylserine with acyl-alkyl residue sum C36:2 |
| PS ae C38:4 | GP.L. | Phosphatidylserine with acyl-alkyl residue sum C38:4 |
| SM C14:0 | S.L. | Sphingomyelin with acyl residue sum C14:0 |
| SM C16:0 | S.L. | Sphingomyelin with acyl residue sum C16:0 |
| SM C16:1 | S.L. | Sphingomyelin with acyl residue sum C16:1 |
| SM C17:0 | S.L. | Sphingomyelin with acyl residue sum C17:0 |
| SM C18:0 | S.L. | Sphingomyelin with acyl residue sum C18:0 |
| SM C18:1 | S.L. | Sphingomyelin with acyl residue sum C18:1 |
| SM C19:0 | S.L. | Sphingomyelin with acyl residue sum C19:0 |
| SM C19:1 | S.L. | Sphingomyelin with acyl residue sum C19:1 |
| SM C19:2 | S.L. | Sphingomyelin with acyl residue sum C19:2 |
| SM C20:0 | S.L. | Sphingomyelin with acyl residue sum C20:0 |
| SM C20:1 | S.L. | Sphingomyelin with acyl residue sum C20:1 |
| SM C20:2 | S.L. | Sphingomyelin with acyl residue sum C20:2 |
| SM C21:0 | S.L. | Sphingomyelin with acyl residue sum C21:0 |
| SM C21:1 | S.L. | Sphingomyelin with acyl residue sum C21:1 |
| SM C21:2 | S.L. | Sphingomyelin with acyl residue sum C21:2 |
| SM C21:3 | S.L. | Sphingomyelin with acyl residue sum C21:3 |
| SM C22:0 | S.L. | Sphingomyelin with acyl residue sum C22:0 |
| SM C22:1 | S.L. | Sphingomyelin with acyl residue sum C22:1 |
| SM C22:2 | S.L. | Sphingomyelin with acyl residue sum C22:2 |
| SM C22:3 | S.L. | Sphingomyelin with acyl residue sum C22:3 |
| SM C23:0 | S.L. | Sphingomyelin with acyl residue sum C23:0 |
| SM C23:1 | S.L. | Sphingomyelin with acyl residue sum C23:1 |
| SM C23:2 | S.L. | Sphingomyelin with acyl residue sum C23:2 |
| SM C23:3 | S.L. | Sphingomyelin with acyl residue sum C23:3 |
| SM C24:0 | S.L. | Sphingomyelin with acyl residue sum C24:0 |
| SM C24:1 | S.L. | Sphingomyelin with acyl residue sum C24:1 |
| SM C24:2 | S.L. | Sphingomyelin with acyl residue sum C24:2 |
| SM C24:3 | S.L. | Sphingomyelin with acyl residue sum C24:3 |
| SM C24:4 | S.L. | Sphingomyelin with acyl residue sum C24:4 |
| SM C26:3 | S.L. | Sphingomyelin with acyl residue sum C26:3 |
| SM C26:4 | S.L. | Sphingomyelin with acyl residue sum C26:4 |
| SM C3:0 | S.L. | Sphingomyelin with acyl residue sum C3:0 |
| lysoPC a C16:0 | GP.L. | Lysophosphatidylcholine with acyl residue sum C16:0 |
| lysoPC a C18:0 | GP.L. | Lysophosphatidylcholine with acyl residue sum C18:0 |
| lysoPC a C18:1 | GP.L. | Lysophosphatidylcholine with acyl residue sum C18:1 |
| lysoPC a C18:2 | GP.L. | Lysophosphatidylcholine with acyl residue sum C18:2 |
| lysoPC a C20:4 | GP.L. | Lysophosphatidylcholine with acyl residue sum C20:4 |
| PC e C18:0 | GP.L. | Lysophosphatidylcholine with alkyl residue sum C18:0 |
| PC aa C30:0 | GP.L. | Phosphatidylcholine with diacyl residue sum C30:0 |
| PC aa C30:1 | GP.L. | Phosphatidylcholine with diacyl residue sum C30:1 |
| PC aa C30:2 | GP.L. | Phosphatidylcholine with diacyl residue sum C30:2 |
| PC aa C32:0 | GP.L. | Phosphatidylcholine with diacyl residue sum C32:0 |
| PC aa C32:1 | GP.L. | Phosphatidylcholine with diacyl residue sum C32:1 |
| PC aa C32:2 | GP.L. | Phosphatidylcholine with diacyl residue sum C32:2 |
| PC aa C34:0 | GP.L. | Phosphatidylcholine with diacyl residue sum C34:0 |
| PC aa C34:1 | GP.L. | Phosphatidylcholine with diacyl residue sum C34:1 |
| PC aa C34:2 | GP.L. | Phosphatidylcholine with diacyl residue sum C34:2 |
| PC aa C34:3 | GP.L. | Phosphatidylcholine with diacyl residue sum C34:3 |
| PC aa C36:0 | GP.L. | Phosphatidylcholine with diacyl residue sum C36:0 |
| PC aa C36:1 | GP.L. | Phosphatidylcholine with diacyl residue sum C36:1 |
| PC aa C36:2 | GP.L. | Phosphatidylcholine with diacyl residue sum C36:2 |
| PC aa C36:3 | GP.L. | Phosphatidylcholine with diacyl residue sum C36:3 |
| PC aa C36:4 | GP.L. | Phosphatidylcholine with diacyl residue sum C36:4 |
| PC aa C36:5 | GP.L. | Phosphatidylcholine with diacyl residue sum C36:5 |
| PC aa C38:1 | GP.L. | Phosphatidylcholine with diacyl residue sum C38:1 |
| PC aa C38:2 | GP.L. | Phosphatidylcholine with diacyl residue sum C38:2 |
| PC aa C38:3 | GP.L. | Phosphatidylcholine with diacyl residue sum C38:3 |
| PC aa C38:4 | GP.L. | Phosphatidylcholine with diacyl residue sum C38:4 |
| PC aa C38:5 | GP.L. | Phosphatidylcholine with diacyl residue sum C38:5 |
| PC aa C38:6 | GP.L. | Phosphatidylcholine with diacyl residue sum C38:6 |
| PC aa C40:4 | GP.L. | Phosphatidylcholine with diacyl residue sum C40:4 |
| PC aa C40:5 | GP.L. | Phosphatidylcholine with diacyl residue sum C40:5 |
| PC aa C40:6 | GP.L. | Phosphatidylcholine with diacyl residue sum C40:6 |
| PC aa C40:7 | GP.L. | Phosphatidylcholine with diacyl residue sum C40:7 |
| PC aa C40:8 | GP.L. | Phosphatidylcholine with diacyl residue sum C40:8 |
| PC ae C32:0 | GP.L. | Phosphatidylcholine with acyl-alkyl residue sum C32:0 |
| PC ae C32:1 | GP.L. | Phosphatidylcholine with acyl-alkyl residue sum C32:1 |
| PC ae C32:6 | GP.L. | Phosphatidylcholine with acyl-alkyl residue sum C32:6 |
| PC ae C34:0 | GP.L. | Phosphatidylcholine with acyl-alkyl residue sum C34:0 |
| PC ae C34:1 | GP.L. | Phosphatidylcholine with acyl-alkyl residue sum C34:1 |
| PC ae C34:2 | GP.L. | Phosphatidylcholine with acyl-alkyl residue sum C34:2 |
| PC ae C34:3 | GP.L. | Phosphatidylcholine with acyl-alkyl residue sum C34:3 |
| PC ae C34:6 | GP.L. | Phosphatidylcholine with acyl-alkyl residue sum C34:6 |
| PC ae C36:1 | GP.L. | Phosphatidylcholine with acyl-alkyl residue sum C36:1 |
| PC ae C36:2 | GP.L. | Phosphatidylcholine with acyl-alkyl residue sum C36:2 |
| PC ae C36:3 | GP.L. | Phosphatidylcholine with acyl-alkyl residue sum C36:3 |
| PC ae C36:4 | GP.L. | Phosphatidylcholine with acyl-alkyl residue sum C36:4 |
| PC ae C36:5 | GP.L. | Phosphatidylcholine with acyl-alkyl residue sum C36:5 |
| PC ae C38:1 | GP.L. | Phosphatidylcholine with acyl-alkyl residue sum C38:1 |
| PC ae C38:2 | GP.L. | Phosphatidylcholine with acyl-alkyl residue sum C38:2 |
| PC ae C38:3 | GP.L. | Phosphatidylcholine with acyl-alkyl residue sum C38:3 |
| PC ae C38:4 | GP.L. | Phosphatidylcholine with acyl-alkyl residue sum C38:4 |
| PC ae C38:5 | GP.L. | Phosphatidylcholine with acyl-alkyl residue sum C38:5 |
| PC ae C38:6 | GP.L. | Phosphatidylcholine with acyl-alkyl residue sum C38:6 |
| PC ae C40:5 | GP.L. | Phosphatidylcholine with acyl-alkyl residue sum C40:5 |
| Histamine | B.Am. | Histamine |
| Serotonin | B.Am. | Serotonin |
| Phenylethyla | B.Am. | Phenylethylamine |
| TXB2 | P.G. | Tromboxane B2 |
| PGF2a | P.G. | Prostaglandin F2alpha |
| 24,25,EPC | O.St. | 24,25-Epoxycholesterol |
| 5B,6B,EPC | O.St. | 5β,6β-Epoxycholesterol |
| 24DHLan | O.St. | 24-Dihydrolanosterol |
| SDMA | B.Am. | Symmetrical Dimethylarginine |
| OH-kynurenine | B.Am. | Hydroxykynurenine |
| 9S-HODE | P.G. | (±)9-hydroxy-10E,12Z-octadecadienoic acid |
| 14(15)-EpETE | P.G. | (±)14(15)-epoxy-5Z,8Z,11Z,17Z-eicosatetraenoic acid |
| 15S-HpETE | P.G. | 15(S)- hydroperoxy-5Z,8Z,11Z,13E-eicosatetraenoic acid |
| 5S-HpETE | P.G. | 5(S)-hydroperoxy-6E,8Z,11Z,14Z-eicosatetraenoic acid |
| LTD4 | P.G. | Leukotriene D4 |
| 6-keto-PGFla | P.G. | 6-keto-Prostaglandin Flalpha |
| 8-isoPGF2a | P.G. | 8-iso-Prostaglandin F2alpha |
| Nitrotyrosine | B.Am. | Nitrotyrosine |
| Dopamine | B.Am. | Dopamine |
| Sarcosine | B.Am. | |
| Cholesterol | O.St. | |

| | | |
|---|---|---|
| Table 1 summarizes analyzed metabolites and respective abbreviations; Glycero-phospholipids are further differentiated with respect to the presence of ester (a) and ether (e) bonds in the glycerol moiety, where two letters (aa, ea, or ee) denote that the first and the second position of the glycerol scaffold are bound to a fatty acid residue, whereas a single letter (a or e) indicates a bond with only one fatty acid residue; e.g. PC_ea_33:1 denotes a plasmalogen phosphatidylcholine with 33 carbons in the two fatty acid side chains and a single double bond in one of them. | | |

### Statistical analysis

All statistical calculations have performed using R [R]. Analytes that were detected in at least 50% of the samples are included resulting in a final list of 221 metabolites.

The concentrations of metabolites in the 72 samples were determined in two analytical batches. A correction factor calculated from the independent QC samples is initially applied to circumvent plate discrepancies before statistical analysis.

The metabolic data is left censored due to the threshold of the mass spectrometer data resulting in non detected peak/signals. Due to a combination of metabolic pathway dynamism, complex sample molecular interaction and overall efficiency of the analytical protocol, replacement of missing data by means of a multivariate algorithm are preferred over a naive imputation by zero. For any statistical treatment, metabolite missing concentrations are replaced by average value of the 6 closest samples to the one where the measurement is missing [KIM] using the function llsImpute in the R package pcaMethods [PCAM].

Except for fold change determination, all data analyses are performed on log basis 2 transformed data.

To test for concentration changes between relapse and non progressive patients for each analyte, a linear model is fitted specifying group and analytical batch using the ImFit function in the R package limma (version 2.16.5) [LIMMA]. Coefficients from the linear model are then combined with equal weight to estimate moderated statistics related to the difference between the two patient cohorts including p value and log 2 fold changes (i.e. log2FC, in the text). Resulting significance levels are presented in form of q values after adjustment by the method described in Benjamini & Hochberg [FDR]. q value threshold of 0.1 is used to pick analytes undergoing to subsequent selection.

Fold changes correspond to the relative change in percent between the median concentration in the Relapse group and the median concentration in the Non Progressive samples. A selection cut off of 20 percent is chosen.

Sensitivity/specificity properties of a classifier comprising one analyte or a combination of analytes are summarized in term of Area Under the Receiver Operating Characteristic Curve (AUC). The function colAUC [CATOOLS] to is used for this purpose and AUC>0.7 is used as selection threshold.

Regardless direction of change, dedicated function vennDiagram in R package limma [LIMMA] is employed to display metabolite classification counts under three univariate techniques: q value, fold change and AUC.

Metabolites selected by at least two methods are making up potential marker composite. Their performance markers are assessed by Diagonal Discriminant Analysis [DLDA] with a contributed sfsmisc library within R [SFSMISC]. Predictive abilities of the models are computed with stratified bootstrap (B=50) strategy were left-out samples are used to compute predicted AUC. Results are presented in form of mean and 10/90 quantiles. For comparison purposes, same strategy is applied to all metabolites (i.e 221) and to the whole set of metabolites without the ones selected in the composite of markers. For graphing, individual ROC curves from the 50 bootstraps are combined by threshold averaging using the function mc.roc in the package FIEmspro [FIEMS]

### Selection of marker combinations

For the set of the biomarkers according to the present invention, parsimonious multi metabolite panels can be used for predicting PCa-relapse. Predictive models using all possible combinations of 2 to 6 markers are built using logistic regression with the dedicated function multinom in the nnet package [NNET] in the R environment. Each model is then further subjected to backward elimination using the Akaike Information Criterion (AIC) to penalize final models having too many parameters. Table 2 provides preferred list of various individual markers and composites from the proposed set of metabolites according to the present invention. While the invention described herein may comprise composite with adequate other than those shown, combination of metabolites would show sensitivities greater than 85% and specificities greater than 75%.

### Results:

In the present invention, metabolic signatures in serum were identified to predict whether a PCa would lead to relapse or not. Thus the sera used in this study were all obtained during the tumour stages of the patients.

Comparisons were performed to generate the data for >2y and >3 y:
(a) Relapsed vs non progressive (patients without relapse for less than 2 years were excluded; only patients without relapse for 2 year or more after radical prostatectomy were included, 28 samples in all)
(b) Relapsed vs non progressive (patients without relapse for less than 3 years were excluded; only patients without relapse for 3 year or more after radical prostatectomy were included, 18 samples in all)

Patients were classified as non progressive (relapse free) based on results from follow up examination after radical prostatectomy. The duration of relapse fee (non progressive) for patients' classification was determined with the last date of follow up examination after treatment.

**Table 2: markers and (exemplary) marker combinations according to the present invention (> 2 years relapse/no relapse; this was also the total group of markers in the >1 year investigation)**

| Table 2A | Ø Relapse free 2 years and more | | | | | |
|---|---|---|---|---|---|---|
| Metabolites | pval | q-value | Auc | log2FC | medFC | |
| PC aa C24:0 | 2,88E-05 | 0,001327 | 0,77 | 0,42 | 21,7 | |
| PC ae C40:3 | 4,61E-08 | 4,24E-06 | 0,74 | 0,54 | 26,9 | |
| PC ae C40:4 | 0,000831 | 0,013047 | 0,66 | 0,32 | 20,2 | |
| lysoPC a C26:0 | 0,000809 | 0,013047 | 0,75 | 0,2 | 11,6 | |
| lysoPC a C6:0 | 7,24E-05 | 0,002222 | 0,74 | 0,4 | 38 | |
| 13S-HODE | 0,000249 | 0,000497 | 0,73 | 1,24 | 166 | |
| 12S-HETE | 0,248173 | 0,310216 | 0,71 | 0,42 | 119,3 | |
| 15S-HETE | 3,07E-07 | 2,73E-06 | 0,77 | 1,49 | 165,5 | |
| LTB4 | 5,33E-06 | 1,78E-05 | 0,76 | 2,19 | 233 | |
| PGE2 | 0,000177 | 0,000443 | 0,69 | 0,72 | 50,1 | |
| PGD2 | 5,45E-07 | 2,73E-06 | 0,81 | 2,72 | 339,5 | |
| 7aOHC | 0,005579 | 0,022316 | 0,67 | 0,72 | 66,5 | |
| 7KC | 9,42E-05 | 0,000565 | 0,74 | 0,66 | 39,7 | |
| 5B,6B,EPC | 2,98E-07 | 3,58E-06 | 0,79 | 1,2 | 180,2 | |
| 5a,6a,EPC | 0,03128 | 0,062559 | 0,64 | 0,42 | 29,9 | |
| 4BOHC | 0,012236 | 0,036707 | 0,73 | 0,37 | 31 | |

| | | | | | | |
|---|---|---|---|---|---|---|
| Table 2A: Avg. relapse free for two years and more | | | | | | |

**Table 2B: preferred marker combinations > 2y**

| Combination | AIC | Accuracy | Sensitivty | Specificty | AUC | Num. of metabolites |
|---|---|---|---|---|---|---|
| lysoPC a C6:0; 5B,6B,EPC; 5a,6a,EPC | 57 | 82 | 85,3 | 77,8 | 90,1 | 3 |
| lysoPC a C26:0; lysoPC a C6:0; 5B,6B,EPC | 61,1 | 80,3 | 85,3 | 74,1 | 86,6 | 3 |
| lysoPC a C6:0; 7aOHC; 5B,6B,EPC | 61,4 | 80,3 | 85,3 | 74,1 | 87,4 | 3 |
| PC aa C24:0; lysoPC a C6:0; 5B,6B,EPC; 5a,6a,EPC | 55,2 | 83,6 | 85,3 | 81,5 | 91,9 | 4 |
| lysoPC a C6:0; 7aOHC; 5B,6B,EPC; 5a,6a,EPC | 55,7 | 86,9 | 91,2 | 81,5 | 90,2 | 4 |
| lysoPC a C6:0; LTB4; 5B,6B,EPC; 5a,6a,EPC | 56,8 | 80,3 | 85,3 | 74,1 | 90,5 | 4 |
| PC ae C40:3; 5B,6B,EPC; 5a,6a,EPC; 4BOHC | 58,9 | 80,3 | 85,3 | 74,1 | 89,8 | 4 |
| PC aa C24:0; lysoPC a C6:0; 7aOHC; 5B,6B,EPC | 59,8 | 83,6 | 85,3 | 81,5 | 90 | 4 |
| lysoPC a C6:0; 15S-HETE; 7aOHC; 5B,6B,EPC | 60,9 | 83,6 | 85,3 | 81,5 | 88,6 | 4 |
| PC aa C24:0; lysoPC a C26:0; lysoPC a C6:0; 5B,6B,EPC | 60,9 | 80,3 | 85,3 | 74,1 | 88,1 | 4 |
| PC aa C24:0; lysoPC a C6:0; 7aOHC; 5B,6B,EPC; 5a,6a,EPC | 53,1 | 90,2 | 94,1 | 85,2 | 93,9 | 5 |
| lysoPC a C6:0; LTB4; 7aOHC; 5B,6B,EPC; 5a,6a,EPC | 53,4 | 85,2 | 91,2 | 77,8 | 93,1 | 5 |
| lysoPC a C6:0; PGE2; 7aOHC; 5B,6B,EPC; 5a,6a,EPC | 54,4 | 82 | 88,2 | 74,1 | 93 | 5 |
| PC ae C40:3; lysoPC a C6:0; 7aOHC; 5B,6B,EPC; 5a,6a,EPC | 54,6 | 86,9 | 91,2 | 81,5 | 93,7 | 5 |
| PC aa C24:0; lysoPC a C26:0; lysoPC a C6:0; 5B,6B,EPC; 5a,6a,EPC | 55,1 | 80,3 | 85,3 | 74,1 | 90,6 | 5 |
| lysoPC a C6:0; 7aOHC; 7KC; 5B,6B,EPC; 5a,6a,EPC | 55,2 | 86,9 | 91,2 | 81,5 | 92,2 | 5 |
| lysoPC a C26:0; lysoPC a C6:0; 7aOHC; 5B,6B,EPC; 5a,6a,EPC | 55,5 | 82 | 88,2 | 74,1 | 92,2 | 5 |
| lysoPC a C6:0; PGD2; 7aOHC; 5B,6B,EPC; 5a,6a,EPC | 55,6 | 85,2 | 88,2 | 81,5 | 92 | 5 |
| lysoPC a C6:0; 12S-HETE; 15S-HETE; 5B,6B,EPC; 5a,6a,EPC | 58,2 | 82 | 85,3 | 77,8 | 91,1 | 5 |
| PC aa C24:0; PC ae C40:4; lysoPC a C26:0; lysoPC a C6:0; 5B,6B,EPC | 60,9 | 80,3 | 85,3 | 74,1 | 89,3 | 5 |
| lysoPC a C6:0; 13S-HODE; LTB4; PGD2; 5a,6a,EPC | 62,5 | 82 | 85,3 | 77,8 | 88,6 | 5 |
| lysoPC a C6:0; 12S-HETE; LTB4; 7aOHC; 5B,6B,EPC; 5a,6a,EPC | 53,3 | 86,9 | 91,2 | 81,5 | 93,8 | 6 |
| lysoPC a C26:0; lysoPC a C6:0; PGD2; 7aOHC; 5B,6B,EPC; 5a,6a,EPC | 55,2 | 83,6 | 85,3 | 81,5 | 92,8 | 6 |
| PC aa C24:0;PC ae C40:4;lysoPC a C26:0;12S-HETE;15S-HETE;5B,6B,EPC | 64,1 | 82 | 85,3 | 77,8 | 88,5 | 6 |

| | | | | | | |
|---|---|---|---|---|---|---|
| Table 2B: preferred marker combinations > 2y | | | | | | |

**Table 3: markers and (exemplary) marker combinations according to the present invention (> 3 years relapse/no relapse)**

| Table 3A: Relapse free 3 years and more | | | | | | |
|---|---|---|---|---|---|---|
| | pval | q-value | auc | log2FC | medFC | |
| PC aa C24:0 | 0,001337 | 0,037199 | 0,77 | 0,43 | 19,7 | |
| PC ae C40:3 | 8,79E-05 | 0,008091 | 0,72 | 0,52 | 21,7 | |
| lysoPC a C26:0 | 0,006707 | 0,088149 | 0,71 | 0,19 | 9,8 | |
| lysoPC a C6:0 | 0,000539 | 0,024783 | 0,73 | 0,41 | 38 | |
| 13S-HODE | 0,006151 | 0,013242 | 0,72 | 1,29 | 196,1 | |
| 12S-HETE | 0,431194 | 0,538992 | 0,74 | 0,31 | 119,3 | |
| 15S-HETE | 2,31E-05 | 0,000194 | 0,76 | 1,52 | 165,5 | |
| LTB4 | 0,000632 | 0,002106 | 0,75 | 2,19 | 233 | |
| PGE2 | 0,006621 | 0,013242 | 0,66 | 0,61 | 38,4 | |
| PGD2 | 3,89E-05 | 0,000194 | 0,85 | 2,99 | 366,3 | |
| 7aOHC | 0,029709 | 0,071303 | 0,67 | 0,72 | 65,6 | |
| 7KC | 0,001064 | 0,006385 | 0,75 | 0,75 | 39,7 | |
| 5B,6B,EPC | 1,8E-07 | 2,16E-06 | 0,86 | 1,46 | 219,4 | |
| 5a,6a,EPC | 0,010567 | 0,042268 | 0,68 | 0,53 | 35,7 | |

| Table 3B: preferred marker combinations > 3y | | | | | | |
|---|---|---|---|---|---|---|
| Combination | AIC | Accuracy | Sensitivty | Specificty | AUC | Num. of metabolites |
| lysoPC a C6:0; 5B,6B,EPC | 42,1 | 88,2 | 94,1 | 76,5 | 91,2 | 2 |
| lysoPC a C6:0; 7aOHC; 5B,6B,EPC | 40 | 88,2 | 91,2 | 82,4 | 93,8 | 3 |
| PC aa C24:0; PC ae C40:3; 5B,6B,EPC | 43,3 | 86,3 | 91,2 | 76,5 | 91,7 | 3 |
| PC ae C40:3; PGD2; 5B,6B,EPC | 44 | 86,3 | 91,2 | 76,5 | 91 | 3 |
| lysoPC a C26:0; PGE2; PGD2 | 44,7 | 82,4 | 85,3 | 76,5 | 91,2 | 3 |
| PC ae C40:3; lysoPC a C26:0; PGD2 | 45,8 | 84,3 | 88,2 | 76,5 | 90,5 | 3 |
| lysoPC a C6:0; 7aOHC; 5B,6B,EPC; 5a,6a,EPC | 34,5 | 90,2 | 94,1 | 82,4 | 96,9 | 4 |
| PC ae C40:3; PGD2; 5B,6B,EPC; 5a,6a,EPC | 39,5 | 86,3 | 91,2 | 76,5 | 93,6 | 4 |
| PC ae C40:3; lysoPC a C26:0; lysoPC a C6:0; PGD2 | 40,1 | 88,2 | 94,1 | 76,5 | 93,8 | 4 |
| PC ae C40:3; 7KC; 5B,6B,EPC; 5a,6a,EPC | 40,3 | 84,3 | 88,2 | 76,5 | 94,1 | 4 |
| PC aa C24:0; PC ae C40:3; lysoPC a C6:0; 5B,6B,EPC | 41,1 | 90,2 | 94,1 | 82,4 | 93,4 | 4 |
| PC ae C40:3; lysoPC a C26:0; lysoPC a C6:0; 5B,6B,EPC | 42,3 | 88,2 | 94,1 | 76,5 | 91,9 | 4 |
| lysoPC a C26:0; lysoPC a C6:0; 13S-HODE; PGD2 | 43,8 | 88,2 | 91,2 | 82,4 | 91,9 | 4 |
| lysoPC a C26:0; lysoPC a C6:0; LTB4; PGD2 | 44 | 86,3 | 91,2 | 76,5 | 91,5 | 4 |
| PC ae C40:3; lysoPC a C26:0; PGE2; PGD2 | 44,1 | 86,3 | 88,2 | 82,4 | 92,7 | 4 |
| PC ae C40:3; lysoPC a C6:0; 13S-HODE; PGD2 | 44,3 | 86,3 | 91,2 | 76,5 | 91,9 | 4 |
| lysoPC a C26:0; PGE2; PGD2; 5a,6a,EPC | 44,6 | 82,4 | 85,3 | 76,5 | 91,7 | 4 |
| lysoPC a C26:0; lysoPC a C6:0; 12S-HETE; PGD2 | 44,8 | 82,4 | 85,3 | 76,5 | 91,2 | 4 |
| PC aa C24:0; PC ae C40:3; lysoPC a C6:0; 5B,6B,EPC; 5a,6a,EPC | 36,4 | 88,2 | 91,2 | 82,4 | 96,7 | 5 |
| PC ae C40:3; lysoPC a C6:0; PGD2; 5B,6B,EPC; 5a,6a,EPC | 38,2 | 88,2 | 91,2 | 82,4 | 95,7 | 5 |
| PC ae C40:3; lysoPC a C26:0; lysoPC a C6:0; PGD2; 5B,6B,EPC38,4 | 88,2 | 94,1 | 76,5 | 95,3 | 5 | |
| lysoPC a C6:0; 13S-HODE; PGD2; 5B,6B,EPC; 5a,6a,EPC | 38,6 | 88,2 | 94,1 | 76,5 | 95,8 | 5 |
| PC ae C40:3; PGD2; 7KC; 5B,6B,EPC; 5a,6a,EPC | 39,1 | 90,2 | 94,1 | 82,4 | 95,3 | 5 |
| PC ae C40:3; lysoPC a C26:0; PGD2; 5B,6B,EPC; 5a,6a,EPC | 39,3 | 88,2 | 91,2 | 82,4 | 94,8 | 5 |
| PC ae C40:3; lysoPC a C26:0; lysoPC a C6:0; 13S-HODE; PGD2 | 39,5 | 90,2 | 94,1 | 82,4 | 93,8 | 5 |
| PC aa C24:0; PC ae C40:3; lysoPC a C26:0; lysoPC a C6:0; 5B,6B,EPC | 40,5 | 90,2 | 94,1 | 82,4 | 94,5 | 5 |
| PC aa C24 0; PC ae C40:3; lysoPC a C6:0; 7aOHC; 5B,6B,EPC | 40,6 | 92,2 | 94,1 | 88,2 | 94,3 | 5 |
| PC ae C40:3; lysoPC a C6:0; 15S-HETE; 7aOHC; 5B,6B,EPC | 41,3 | 86,3 | 88,2 | 82,4 | 94,6 | 5 |
| lysoPC a C26:0; lysoPC a C6:0; 13S-HODE; PGD2; 5a,6a,EPC | 42,6 | 84,3 | 88,2 | 76,5 | 93,3 | 5 |
| PC ae C40:3; lysoPC a C26:0; 13S-HODE; PGD2; 5a,6a,EPC | 43,5 | 86,3 | 91,2 | 76,5 | 92,4 | 5 |
| lysoPC a C26:0; lysoPC a C6:0; 12S-HETE; PGD2; 5a,6a,EPC | 43,5 | 82,4 | 85,3 | 76,5 | 92,6 | 5 |
| lysoPC a C26:0; lysoPC a C6:0; LTB4; PGD2; 5a,6a,EPC | 43,6 | 86,3 | 91,2 | 76,5 | 92,2 | 5 |
| lysoPC a C26:0; 13S-HODE; PGE2; PGD2; 5a,6a,EPC | 43,6 | 86,3 | 91,2 | 76,5 | 93,4 | 5 |
| PC ae C40:3; lysoPC a C26:0; PGE2; PGD2; 7KC | 43,8 | 88,2 | 88,2 | 88,2 | 92,9 | 5 |
| lysoPC a C26:0; lysoPC a C6:0; PGE2; PGD2; 5a,6a,EPC | 44 | 86,3 | 88,2 | 82,4 | 92,2 | 5 |
| PC ae C40:3; 13S-HODE; PGD2; 7KC; 5a,6a,EPC | 44 | 88,2 | 91,2 | 82,4 | 92,2 | 5 |
| 15S-HETE; LTB4; PGE2; PGD2; 7KC | 47,4 | 88,2 | 94,1 | 76,5 | 91,9 | 5 |
| PC aa C24:0;PCaeC40:3;lysoPCaC26:0;lysoPCaC6:0;5B,6B,EPC;5a,6a,EPC | 33,2 | 92,2 | 97,1 | 82,4 | 98,4 | 6 |
| PC ae C40:3;lysoPC a C26:0;lysoPC a C6:0;PGD2;5B,6B,EPC;5a,6a,EPC | 33,4 | 92,2 | 94,1 | 88,2 | 97,1 | 6 |
| PC aa C24:0;PC ae C40:3;lysoPC a C6:0; 7aOHC;5B,6B,EPC;5a,6a,EPC | 35,5 | 94,1 | 94,1 | 94,1 | 97,1 | 6 |
| lysoPC a C26:0; lysoPC a C6:0; 12S-HETE; PGD2; 5B,6B,EPC; 5a,6a,EPC | 37 | 90,2 | 91,2 | 88,2 | 95,7 | 6 |
| lysoPC a C26:0; lysoPC a C6:0; 15S-HETE; PGD2; 5B,6B,EPC; 5a,6a,EPC | 37,7 | 88,2 | 91,2 | 82,4 | 95,7 | 6 |
| PC ae C40:3; lysoPC a C26:0; lysoPC a C6:0; PGD2; 7aOHC; 5B,6B,EPC | 37,7 | 90,2 | 94,1 | 82,4 | 95,8 | 6 |
| lysoPC a C26:0; lysoPC a C6:0; LTB4; PGD2; 5B,6B,EPC; 5a,6a,EPC | 38,6 | 88,2 | 91,2 | 82,4 | 96,2 | 6 |
| PC ae C40:3; lysoPC a C26:0; PGD2; 7KC; 5B,6B,EPC; 5a,6a,EPC | 38,7 | 88,2 | 91,2 | 82,4 | 95,3 | 6 |
| PC ae C40:3; lysoPC a C26:0; lysoPC a C6:0;135-HODE;PGD2;5a,6a,EPC | 39 | 88,2 | 91,2 | 82,4 | 95,3 | 6 |
| PC ae C40:3; lysoPC a C26:0; lysoPC a C6:0;125-HETE;15S-HETE;PGD2 | 39,6 | 88,2 | 91,2 | 82,4 | 95,8 | 6 |
| PC ae C40:3; lysoPC a C26:0; lysoPC C6:0; 6a,EPC | 39,8 | 86,3 | 91,2 | 76,5 | 95 | 6 |
| PC ae C40:3; lysoPC a C26:0; lysoPC a C6:0; PGD2; 7aOHC; 7KC | 40,2 | 88,2 | 94,1 | 76,5 | 94,8 | 6 |
| PC aa C24:0;PC ae C40:3;lysoPC a C26:0;lysoPCaC6:0;7aOHC;5B,6B,EPC | 40,4 | 92,2 | 94,1 | 88,2 | 95,7 | 6 |
| PC ae C40:3; lysoPC a C26:0; lysoPC a C6:0; LTB4; 7aOHC; 5B,6B,EPC | 41,3 | 88,2 | 94,1 | 76,5 | 95,2 | 6 |
| PC ae C40:3;lysoPCaC26:0;lysoPCaC6:0;12S-HETE;15S-HETE;5B,6B,EPC | 41,4 | 88,2 | 94,1 | 76,5 | 94,6 | 6 |
| PC aa C24:0;PC ae C40:3;lysoPC a C6:0;125-HETE;15S-HETE;5B,6B,EPC | 41,6 | 92,2 | 94,1 | 88,2 | 95,2 | 6 |
| lysoPC a C26:0; lysoPC a C6:0; 13S-HODE; PGE2; PGD2; 5a,6a,EPC | 42,4 | 90,2 | 91,2 | 88,2 | 93,3 | 6 |
| lysoPC a C26:0; lysoPC a C6:0; 12S-HETE; PGE2; PGD2; 5a,6a,EPC | 43,2 | 88,2 | 91,2 | 82,4 | 93,8 | 6 |
| PC ae C40:3; lysoPC a C6:0; 13S-HODE; 15S-HETE; PGD2; 5B,6B,EPC | 43,3 | 90,2 | 97,1 | 76,5 | 94,5 | 6 |
| PC ae C40:3; 13S-HODE; PGE2; PGD2; 7KC; 5a,6a,EPC 43,4 | 88,2 | 91,2 | 82,4 | 93,3 | 6 | |
| PC ae C40:3; lysoPC a C26:0; PGE2; PGD2; 7KC; 5a,6a,EPC | 43,5 | 88,2 | 88,2 | 88,2 | 93,9 | 6 |
| PC ae C40:3; lysoPC a C6:0; 13S-HODE; 12S-HETE; 15S-HETE; PGD2 | 44,5 | 86,3 | 88,2 | 82,4 | 92,7 | 6 |
| lysoPC a C26:0; lysoPC a C6:0; 12S-HETE; 15S-HETE; LTB4; PGD2 | 44,7 | 86,3 | 91,2 | 76,5 | 93,1 | 6 |
| PC ae C40:3; 13S-HODE; 12S-HETE; 15S-HETE; PGD2; 7KC | 45 | 86,3 | 91,2 | 76,5 | 92,6 | 6 |
| PC ae C40:3; 13S-HODE; 15S-HETE; PGE2; PGD2; 7KC | 45,2 | 90,2 | 94,1 | 82,4 | 93,8 | 6 |
| PC ae C40:3; lysoPC a C26:0; 13S-HODE; 12S-HETE; 15S-HETE; PGD2 | 45,2 | 82,4 | 85,3 | 76,5 | 92,7 | 6 |
| PC ae C40:3; lysoPC a C26:0; 12S-HETE; PGD2; 7KC; 5a,6a,EPC45,6 | 84,3 | 88,2 | 76,5 | 92,2 | 6 | |
| PC ae C40:3; lysoPC a C26:0; 12S-HETE; 15S-HETE; PGD2; 7KC | 45,7 | 82,4 | 85,3 | 76,5 | 92,9 | 6 |
| PC ae C40:3; 15S-HETE; LTB4; PGE2; PGD2; 7KC | 47 | 88,2 | 91,2 | 82,4 | 92,6 | 6 |
| PC ae C40:3; 12S-HETE; 15S-HETE; PGE2; PGD2; 7KC | 47,3 | 82,4 | 85,3 | 76,5 | 92,4 | 6 |
| PC ae C40:3; lysoPC a C6:0; 15S-HETE; LTB4; PGD2; 7KC | 47,8 | 84,3 | 88,2 | 76,5 | 91,3 | 6 |
| PC ae C40:3; lysoPC a C6:0; 12S-HETE; 15S-HETE; 7aOHC; 7KC | 50,7 | 82,4 | 85,3 | 76,5 | 90 | 6 |

| | | | | | | |
|---|---|---|---|---|---|---|
| Table 3B: preferred marker combinations > 3y | | | | | | |

p value, q value etc... are general terms that will be understood by anyone in the field. Exact definitions are:
"p value": probability of obtaining a result greater than the observed quantity assuming that this result is obtained by chance in statistical hypothesis testing
"q value": minimum expected false positive rate at which the test may be called significant
"sensitivity": proportion of positive examples which are correctly identified as positive in a binary classification test
"specificity": proportion of negative examples which are correctly identified as negative in a binary classification test
"accuracy": proportion of true results (both true positives and true negatives) in the population
"AUC": area under the receiver operating characteristic (ROC) curve. ROC curve is a graphical representation of the relationship of the sensitivity and (1 - specificity) across all possible threshold values that define the decision boundaries of a binary classifier.
"fold change": Fold change is a mathematical operation describing how much two groups differ.
"FC(log2)": is defined as the difference between the group means calculated from the log basis 2 transformed data
"medFC": is defined as the ratio between the group medians minus 1
"AIC": Akaike Information Criterion is a measure of the goodness of fit of an estimated statistical model. In the general case, AIC = 2k - 2*ln(L), where k is the number of parameters in the statistical model, and L is the maximized value of the likelihood function for the estimated model.

Those skilled in the art would understand that each assay of the multi anaylte panel needs to undergo fit for purpose assay.

Those skilled in the art would understand that a suitable classification algorithm could also comprise (diagonal) linear or quadratic discriminant analysis (LDA, QDA, DLDA, DQDA), perceptron, shrunken centroids, regularized discriminant analysis (RDA), random forests (RF), neural networks (NN), Bayesian networks, hidden Markov models, support vector machines (SVM), generalized partial least squares (GPLS), inductive logic programming (ILP), generalized additive models, gaussian processes, regularized least square regression, recursive partitioning and regression trees, K-nearest neighbour classifiers (K-NN), fuzzy classifiers, bagging, boosting, and naive Bayes.

### References:

[R] R Development Core Team (2009). R: A language and environment for statistical computing. R Foundation for Statistical Computing, Vienna, Austria. ISBN 3-900051-07-0, URL http://www.R-project.org ORGINAL SOFTWARE WEBPAGE
[KIM] H. Kim, G.H. Golub and H. Park (2005) Missing value estimation for DNA microarray gene expression data: local least squares imputation, Bioinformatics, 21(2):187-198.
[PCAM] W. Stacklies and H. Redestig (2007) pcaMethods: A collection of PCA methods, R package version 1.18.0 (ONLINE DOCUMENT)
[LIMMA] G.K. Smyth (2005). Limma: linear models for microarray data. In: 'Bioinformatics and Computational Biology Solutions using R and Bioconductor'. R. Gentleman, V. Carey, S. Dudoit, R. Irizarry, W. Huber (eds), Springer, New York, 397-420.
[FDR] Y. Benjamini and Y. Hochberg (1995) Controlling the false discovery rate: a practical and powerful approach to multiple testing, Journal of the Royal Statistical Society Series B, 57, 289-300
[DLDA] S. Dudoit, J. Fridlyand, and T. P. Speed. (2000) Comparison of Discrimination Methods for the Classification of Tumors Using Gene Expression Data. (Statistics, UC Berkeley, June 2000, Tech Report #576)
[SFSMISC] M. Maechler and many others. (2009) sfsmisc: Utilities from Seminar fuer Statistik ETH Zurich, R package version 1.0-8
[CATOOLS] Tuszynski J. (2008) caTools: Tools: moving window statistics, GIF, Base64, ROC AUC, etc., R package version 1.9 ONLINE DOCUMENT
[FIEMS] M. Beckmann, D. Enot and W. Lin (2007) FIEmspro: Flow Injection Electrospray Mass Spectrometry Processing: \\ data processing, classification modelling and variable selection in metabolite fingerprinting, R package version 1.1-0
[NNET] Venables, W. N. & Ripley, B. D. (2002) Modern Applied Statistics with S. Fourth Edition. Springer, New York. ISBN 0-387-95457-0

## Claims

1. : The use of at least one substance selected from the group consisting of Phosphatidylcholine with diacyl residue sum C24:0 (PC aa C24:0); Phosphatidylcholine with diacyl residue sum C40:3 (PC ae C40:3); Phosphatidylcholine with diacyl residue sum C40:4 (PC ae C40:4); Lysophosphatidylcholine with acyl residue sum C26:0 (lysoPC a C26:0); Lysophosphatidylcholine with acyl residue sum C6:0 (lysoPC a C6:0); 13(S)-hydroxy-9Z,11E-octadecadienoic acid (13S-HODE); 12(S)-hydroxy-5Z,8Z,10E,14Z-eicosatetraenoic acid (12S-HETE); 15(S)-hydroxy-5Z,8Z,11Z,13E-eicosatetraenoic acid (15S-HETE); Leukotriene B4 (LTB4); Prostaglandin E2 (PGE2); Prostaglandin D2 (PGD2); 7α-Hydroxycholesterol (7aOHC); 7-Ketocholesterol (7KC); 5β,6β-Epoxycholesterol (5b,6b,EPC); 5α,6α-Epoxycholesterol (5a,6a,EPC); and 4β-Hydroxycholesterol (4BOHC); for prognosing relapse of a prostate cancer (PCa) in a sample of a body fluid or a tissue sample of a PCa patient.

2. : A method for prognosing relapse of a prostate cancer (PCa) patient in a sample of a body fluid or a tissue sample of said PCa patient **characterised in that** at least one one substance selected from the group consisting of Phosphatidylcholine with diacyl residue sum C24:0 (PC aa C24:0); Phosphatidylcholine with diacyl residue sum C40:3 (PC ae C40:3); Phosphatidylcholine with diacyl residue sum C40:4 (PC ae C40:4); Lysophosphatidylcholine with acyl residue sum C26:0 (lysoPC a C26:0); Lysophosphatidylcholine with acyl residue sum C6:0 (lysoPC a C6:0); 13(S)-hydroxy-9Z,11E-octadecadienoic acid (13S-HODE); 12(S)-hydroxy-5Z,8Z,10E,14Z-eicosatetraenoic acid (12S-HETE); 15(S)-hydroxy-5Z,8Z,11Z,13E-eicosatetraenoic acid (15S-HETE); Leukotriene B4 (LTB4); Prostaglandin E2 (PGE2); Prostaglandin D2 (PGD2); 7α-Hydroxycholesterol (7aOHC); 7-Ketocholesterol (7KC); 5β,6β-Epoxycholesterol (5b,6b,EPC); 5α,6α-Epoxycholesterol (5a,6a,EPC); and 4β-Hydroxycholesterol (4BOHC) is detected and quantified in said sample and the result of this quantification is compared with the amount of the at least one detected and quantified substance in a corresponding sample of known relapse status.

3. : Method according to claim 2, **characterised in that** the sample is a blood or blood derived sample or a urine sample, preferably a blood, plasma or serum sample.

4. : Method according to claim 2 or 3, **characterised in that** the amount of the at least one detected and quantified substance in a corresponding sample of known relapse status is a numerical limit for the amount of this substance.

5. : Method according to any one of claim 2 to 4, **characterised in that** the substance is detected and quantified by mass spectroscopy, preferably liquid chromatography mass spectroscopy (LC-MS), especially high performance liquid chromatography mass spectroscopy (HPLC-MS) or reverse phase liquid chromatography mass spectroscopy (RPLC-MS); electro-spray ionisation mass spectroscopy (ESI-MS), gas chromatography mass spectroscopy (GC-MS), atmospheric pressure chemical ionisation mass spectroscopy (APCI-MS), capillary electrophoresis mass spectroscopy (CE-MS), tandem mass spectroscopy (MS-MS); or combinations of these mass spectroscopy techniques.

6. : Method according to any one of claim 2 to 5, **characterised in that** at least two, preferably at least three, especially at least four of the substances are determined, quantified and compared.

7. : Method according to any one of claim 2 to 6, **characterised in that** the amount of the at least one detected and quantified substance in a corresponding sample of known relapse status is the amount of this substance in a sample of a PCa relapse patient or the average amount of this substance of a pool of samples of PCa relapse patients.

8. : Method according to any one of claim 2 to 7, **characterised in that** the sample is regarded as having a prognosis for PCa relapse, if the at least one substance is contained in an amount which is at least 10%, preferably at least 20%, more preferably at least 50 %, even more preferred at least 100%, especially at least 200%, increased compared to the amount of this substance in a sample which does not have a prognosis for PCa relapse.

9. : Method according to any one of claim 2 to 8, **characterised in that** the comparison is performed using software-based statistical and bioinformatic data analyses.

10. : Use of a mass spectrometer for carrying out the method according to any one of claims 2 to 9.

11. : Kit for carrying out the method according to any one of claims 2 to 9 comprising
- a mass spectrometer,
- a standard sample containing a known amount of at least one substance from the group according to claim 2 and
- a sample of a PCa patient containing an unknown amount of at least one substance from the group according to claim 2.

12. : Kit according to claim 11, **characterised in that** it further comprises one or more of the following components: quality control samples, analyte standards, internal standards or data analysis software.
